# FASCICULE DE BREVET EUROPEEN

(11) **EP 0 658 553 B1**
(45) Date de publication et mention de la délivrance du brevet: **04.11.1998**
(21) Numéro de dépôt: 94402553.5
(22) Date de dépôt: 10.11.1994
(51) Int. Cl.: C07D 333/38, C07D 333/40, C07D 213/80, C07D 207/40, C07C 69/94, C07C 69/78, C07C 65/17

(54) **Composés aromatiques polycycliques, compositions pharmaceutiques et cosmétiques les contenant et utilisations**
Polyzyklische aromatische Verbindungen, sie enthaltende pharmazeutische und kosmetische Zusammensetzungen und deren Verwendungen
Polycyclic aromatic compounds, pharmaceutical and cosmetic compositions containing them and uses thereof

(30) Priorité: 15.12.1993 FR 9315068
(43) Date de publication de la demande: 21.06.1995
(73) Titulaire: CENTRE INTERNATIONAL DE RECHERCHES DERMATOLOGIQUES GALDERMA, ( CIRD GALDERMA), 06560 Valbonne (FR)
(72) Inventeur: Charpentier, Bruno, F-06410 Biot (FR); Bernardon, Jean-Michel, F-06650 Le Rouret (FR)
(74) Mandataire: Tezier Herman, Béatrice

(56) Documents cités:
- EP-A- 0 210 929
- WO-A-92/06948
- WO-A-92/19583
- BULLETIN DE LA SOCIETE CHIMIQUE DE FRANCE., no.7-8, 1973, PARIS FR pages 2384 - 2388 H. EUSTATHOPOULOS ET AL. 'No. 425. - Phénylation des diméthyl naphthalènes symétriques. III. - Synthèses et identifications des isomères phénylés'
- CHEMICAL ABSTRACTS, vol. 55, no. 26, 25 Décembre 1961, Columbus, Ohio, US; abstract no. 27238g, N.G. SIDOROVA ET AL. 'Cycloalkylation of aromatic compounds. XVII. Cyclohexylation of phenanthrene and anthracene.'

## Description

L'invention concerne, à titre de produits industriels nouveaux et utiles, des composés aromatiques polycycliques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photo-induit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

La demanderesse a proposé par le passé des composés présentant des activités similaires mais dont les structures sont différentes des composés objets de la présente demande. On citera par exemple les composés décrits dans les demandes de brevet EP-A-210 929 ou WO-A-92/06948.

Les composés selon l'invention peuvent être représentés par la formule générale (I) suivante : 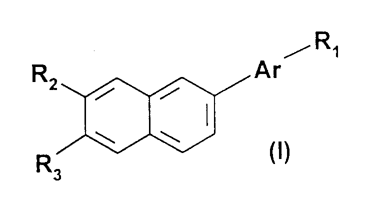 dans laquelle :
* R₁ représente :
   (i) un atome d'hydrogène
   (ii) un radical -CH₃
   (iii) un radical -CH₂OH
   (iv) un radical -O-R₄
   (v) un radical -S(O)t-R₅
   (vi) un radical -CO-R₆
   R₄, R₅, R₆ et t ayant la signification donnée ci-après,
* Ar représente un radical choisi parmi les radicaux de formules (a)-(i) suivantes : 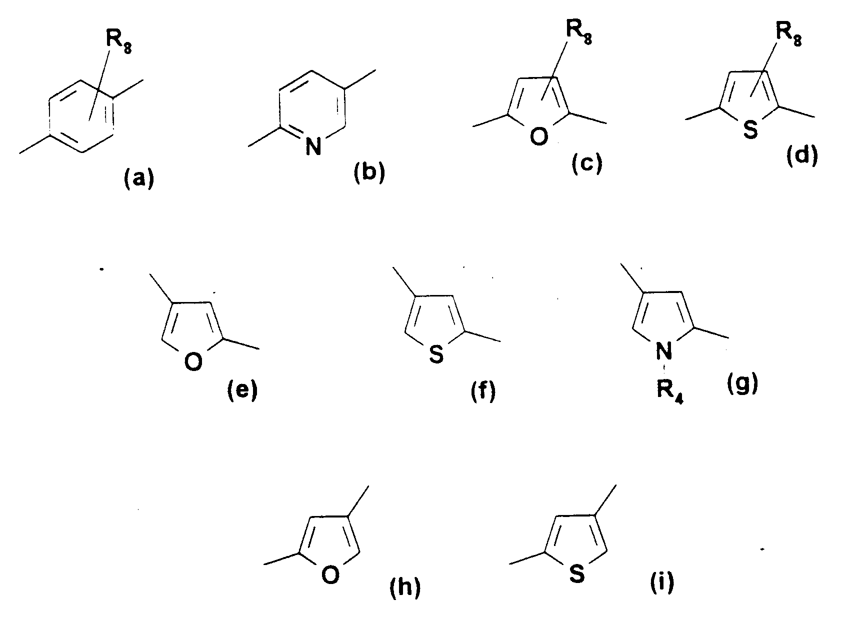 R₄ et R₈ ayant la signification donnée ci-après,
* R₂ représente un radical alkyle linéaire ou ramifié ayant de 1 à 20 atomes de carbone ou un radical cycloaliphatique,
* R₃ représente :
   (a) un radical -X-(CH₂)ₘ-R₁₀
   (b) un radical -(CH₂)ₘ-R₁₁
   (c) un radical -CH=CH-(CH₂)ₙ-R₁₁
   (d) un radical -O-CH₂-O-CH₂-CH₂-O-CH₃
X, R₁₀, R₁₁, n et m ayant la signification donnée ci-après, étant entendu que dans tout ce qui précède :
- R₂ et R₃, pris ensemble, peuvent former avec le noyau naphtalénique adjacent un cycle à 5 ou 6 chainons éventuellement substitué par des groupes méthyle et/ou éventuellement interrompu par un atome d'oxygène ou par un radical -S(O)z-, z ayant la signification donnée ci-après,
- R₄ représente un atome d'hydrogène, un radical alkyle inférieur ou un radical -(CH2)p-(CO)q-R₅, p, q et R5 ayant la signification donnée ci-après,
- R₅ représente un radical alkyle inférieur ou un hétérocycle,
- R₆ représente :
   (a) un atome d'hydrogène
   (b) un radical alkyle inférieur
   (c) un radical de formule : 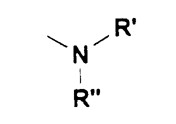 R' et R'' ayant la signification donnée ci-après,
   (d) un radical -O-R₇, R₇ ayant la signification donnée ci-après,
- R₇ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre ou un reste d'amino acide ou de peptide,
- R₈ représente un atome d'halogène, un radical alkyle inférieur, un radical hydroxy, un radical -OR₉ ou -O-COR₉, R₉ ayant la signification donnée ci-après, ou encore un atome d'hydrogène lorsque R₂ est le radical 1-adamantyl et R₃ est différent du radical -OCH₃ ou du radical -OH,
- R₉ représente un radical alkyle inférieur,
- R₁₀ représente un atome d'hydrogène, un radical alkyle inférieur, un radical aryle, un radical aralkyle, un radical monohydroxyalkyle, un radical polyhydroxyalkyle, un radical -CO-R₆ ou bien encore, mais seulement dans le cas où m est supérieur ou égal à 2, un radical de formule : 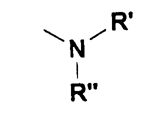 R' et R'' ayant la signification donnée ci-après,
- R₁₁ représente un atome d'hydrogène, un radical monohydroxyalkyle, un radical polyhydroxyalkyle, un radical -CO-R₆, un radical de formule : 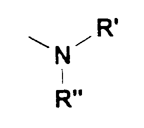 R' et R'' ayant la signification donnée ci-après,
   ou bien encore, mais seulement dans le cas où m est supérieur ou égal à 1, un radical hydroxy, un radical -OR₉ ou un radical -O-COR₉,
- R' et R'' représentent un atome d'hydrogène, un radical alkyle inférieur, un radical mono ou polyhydroxyalkyle, un radical aryle éventuellement substitué ou un reste d'amino acide ou de sucre ou encore, pris ensemble, forment un hétérocycle,
- X représente un atome d'oxygène ou de soufre,
- n est un nombre entier compris inclusivement entre 0 et 4,
- m est un nombre entier compris inclusivement entre 0 et 6,
- p est un nombre entier compris inclusivement entre 1 et 3,
- q est un nombre entier compris inclusivement entre 0 et 1,
- t est un nombre entier compris inclusivement entre 0 et 2,
- z est un nombre entier compris inclusivement entre 0 et 2.

L'invention vise également les sels des composés de formule (I) ci-dessus dans le cas où les radicaux R₁ ou R₁₀ ou R₁₁ représentent une fonction acide carboxylique, ou bien encore lorsque R₁₀ ou R₁₁ représentent une fonction amine, ainsi que les analogues chiraux desdits composés de formule (I). Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle. hexadécyle et octadécyl.

Par radical cycloaliphatique. on entend notamment un radical mono ou polycyclique tel que plus particulièrement le radical 1-méthyl cyclohexyle et 1-adamantyle.

Par radical monohydroxyalkyle. on entend un radical ayant de préférence 2 ou 3 atomes de carbone. notamment un radical 2-hydroxyéthyle, 2-hydroxypropyle ou 3-hydroxypropyle.

Par radical polyhydroxyalkyle. on entend un radical contenant de préférence de 3 à 6 atomes de carbone et de 2 à 5 groupes hydroxyles tels que les radicaux 2,3-dihydroxypropyle. 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle. on entend de préférence le radical benzyle ou phénéthyle eventuellement substitué par au moins un atome d'halogène. un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque R₈ représente un atome d'halogéne, celui-ci est de préférence un atome de fluor, de chlore et de brome.

Parmi les composés de formule (I) ci-dessus rentrant dans le cadre de la présente invention, on peut notamment citer les suivants :
- 2-(5,6,7,8-tétrahydro-5,5,8,8-tétraméthyl-2-anthracényl)-4-thiophène carboxylate d'éthyle,
- Acide 2-(5,6,7,8-tétrahydro-5,5,8,8-tétraméthyl-2-anthracényl)-4-thiophène carboxylique,
- 4-(5,6,7,8-tétrahydro-5,5,8,8-tétraméthyl-2-anthracényl)-2-thiophène carboxylate de méthyle,
- Acide 4-(5,6,7,8-tétrahydro-5,5,8,8-tétraméthyl-2-anthracényl)-2-thiophène carboxylique,
- 6-(5,6,7,8-tétrahydro-5,5,8,8-tétraméthyl-2-anthracényl)-nicotinate d'éthyle,
- Acide 6-(5,6,7,8-tétrahydro-5,5,8,8-tétraméthyl-2-anthracényl)-nicotinique.
- Acide N-méthyl-4-(5,6,7,8-tétrahydro-5,5,8,8-tétraméthyl-2-anthracényl)-2-pyrrole carboxylique.
- Acide 4-(5,6,7,8-tétrahydro-5,5,8,8-tétraméthyl-2-anthracényl)-2-pyrrole carboxylique.
- 2-hydroxy-4-(5,6,7,8-tétrahydro-5,5,8,8-tétraméthyl-2-anthracényl)benzoate de méthyle.
- Acide 2-hydroxy-4-(5,6,7,8-tétrahydro-5,5,8,8-tétraméthyl-2-anthracényl)benzoïque.
- Acide 2-méthoxy-4-(5,6,7,8-tétrahydro-5,5,8,8-tétraméthyl-2-anthracényl)benzoïque.
- Acide 2-hydroxy-4-[7-(1-adamantyl)-6-benzyloxy-2-naphtyl]benzoïque.
- Acide 2-hydroxy-4-[7-(1-adamantyl)-6-hydroxy-2-naphtyl]benzoïque.
- Acide 2-hydroxy-4-[7-(1-adamantyl)-6-hexyloxy-2-naphtyl]benzoïque.
- Acide 4-[7-(1-adamantyl)-6-méthoxyéthoxyméthoxy-2-naphtyl]benzoïque.
- Acide 3-méthyl-4-(5,6,7,8-tétrahydro-5,5,8,8-tétraméthyl-2-anthracényl)benzoïque.
- Acide N-propyl-4-(5,6,7,8-tétrahydro-5,5,8,8-tétraméthyl-2-anthracényl)-2-pyrrole carboxylique.
- Acide 2-propyloxy-4-(5,6,7,8-tétrahydro-5,5,8,8-tétraméthyl-2-anthracényl) benzoïque.
- Acide 2-hexyloxy-4-(5,6,7,8-tétrahydro-5,5,8,8-tétraméthyl-2-anthracényl) benzoïque.
- Acide 5-[7-(1-adamantyl)-6-benzyloxy-2-naphtyl]-2-thiophènecarboxylique.
- Acide 4-[7-(1-adamantyl)-6-benzyloxy-2-napntyl]benzoïque.
- Acide 2-chloro-4-(5,6,7,8-tétrahydro-5,5,8,8-tétraméthyl-2-anthracényl) benzoïque.
- Acide 2-hydroxy-4-[7-(1-adamantyl)-6-(4-fluorobenzyl)oxy-2-naphtyl]benzoïque.
- Acide 2-[7-(1-adamantyl)-6-hydroxy-2-naphtyl]-4-thiophènecarboxylique.
- Acide 2-(7-(1-adamantyl)-6-méthoxy-2-naphtyl]-4-thiopnènecarboxylique.
- 4-[7-(1-adamantyl)-6-benzyloxycarbonyl-2-naphtyl]benzoate de méthyle.

Selon la présente invention, les composés de formule (I) plus particuliérement préférés sont ceux pour lesquels Ar représente un radical de formule (a) ou (i), R₆ représente un radical -OR₇, R₇ ayant la signification donnée ci-avant, et R₂ et R₃ pris ensemble forment avec le noyau naphtalénique adjacent un cycle à 6 chaînons substitué par des groupes méthyles.

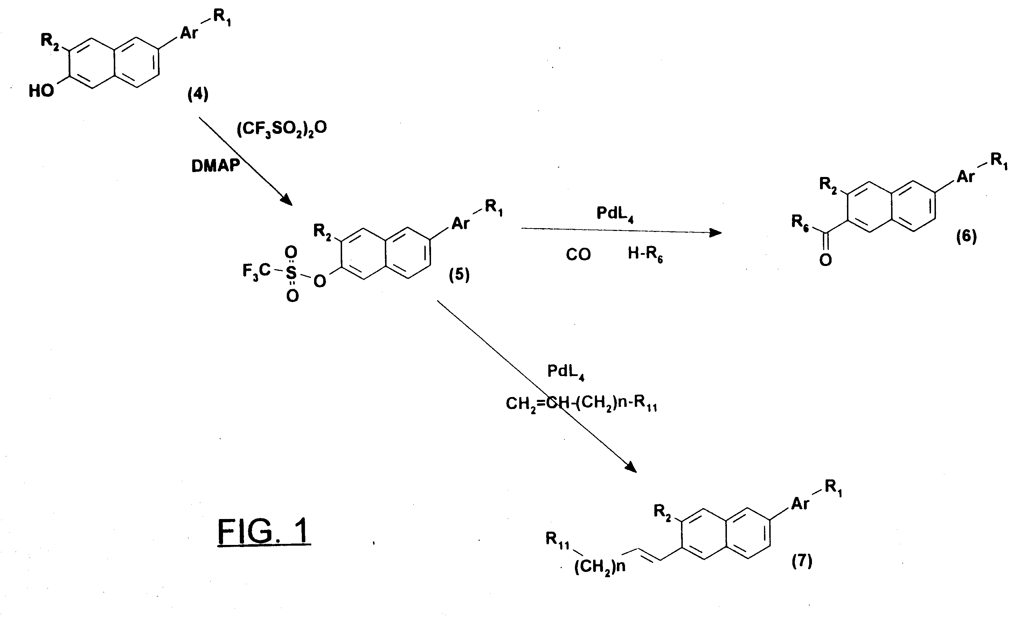

La présente invention a également pour objet les procédés de préparation des composés de formule (I), en particulier selon les schémas réactionnels donnés ci-dessous et à la Figure 1.

Les composés de formule (I) peuvent ainsi être obtenus :
- soit par une réaction de couplage entre un dérivé halogéné (1) et un dérivé halogéné (2): 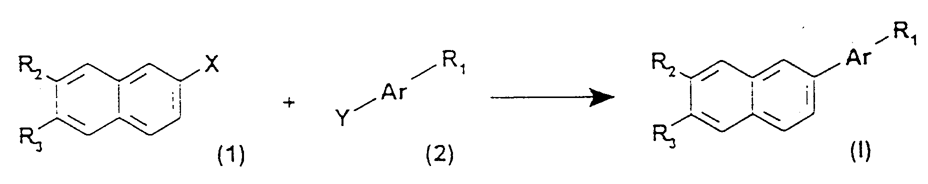 X et Y représentant un atome de chlore, de brome ou d'iode dans un premier temps, l'halogénure (1) est converti en un lithien ou magnésien puis zincique et est couplé au dérivé (2) en présence d'un catalyseur an nickel ou au palladium, selon les conditions de couplage biaryl décrites par E. Negishi et al., dans J. Org. Chem. (1977), 42, 1821.
- soit par une réaction de couplage entre un acide boronique (3) et un dérivé halogéné (2) : 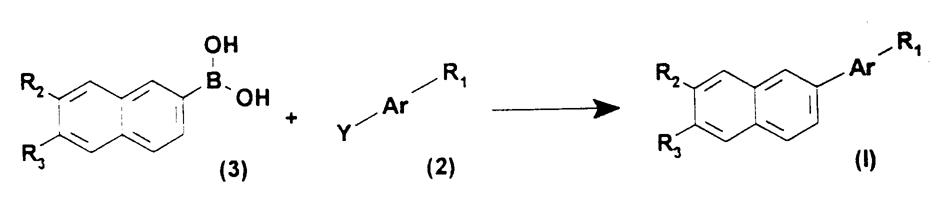 la réaction de couplage étant éffectuée en présence d'un catalyseur au palladium, par exemple le tétrakis(triphénylphosphine)palladium selon les conditions décrites par N. Miyaura et al., dans Synthetic Communications (1981) 11(7), 513-519 ; le dérivé acide boronique (3) peut être lui-même obtenu par exemple à partir du dérivé halogéné (1) par transformation en lithien puis réaction avec le triméthyl borate et hydrolyse.

Dans les formules et réactions ci-dessus, R₁, R₃, R₈ ont les mêmes significations que celles données ci-avant pour la formule générale (I) ou en sont des dérivés convenablement protégés pour être compatibles avec les conditions de couplage. En particulier, le substituant R₃ est un phénol protégé sous forme de tert-butyldiméthylsililoxy ou un radical alcoxy.

Lorsque R₃ est un radical-(CH2)m-CO-R₆ ou un radical-CH=CH-(CH2)n-R₁₁ les composés sont préparés préferentiellement à partir du dérivé phénolique (4) selon le schéma réactionnel donné à la Figure 1, c'est à dire conversion du dérivé phénolique (4) en triflate (5) puis substitution nucléophile en présence d'un catalyseur au palladium selon les conditions générales décrites par S. Cacchi et al., dans Tetrahedron Letter 1986, 27, 3931-3934, ou par W. J. SCOTT et al., dans J. Org. Chem. 1985, 50, 2302-2308.

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités des composés respectivement dans les domaines de la différenciation et de la prolifération cellulaire.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée telle que l'eczéma ou l'atopie respiratoire ou encore l'hypertrophie gingivale les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, ou un de ses sels ou l'un de ses analogues chiraux.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses analogues chiraux ou encore l'un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphéres ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses analogues chiraux ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses analogues chiraux ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoides et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### 2-(5,6,7,8-tétrahydro-5,5,8,8-tétraméthyl-2-anthracényl)-4-thiophène carboxylate d'éthyle

A une suspension de 724 mg (30 mmoles) de magnésium dans 10 ml de THF, on ajoute goutte à goutte une solution de 9,5 g (30 mmoles) de 5,6,7,8-tétrahydro-5,5,8,8-tétraméthyl-2-bromoanthracène. Une fois l'addition terminée, on chauffe à reflux pendant une heure. A température ambiante, on ajoute 4,1 g (30 mmoles) de chlorure de Zinc anhydre et agite pendant une heure. On ajoute ensuite sucessivement 5,2 g (22 mmoles) de 2-bromo-4-thiophènecarboxylate d'éthyle et 120 mg (0,22 mmoles) du complexe NiCl₂/DPPE et laisse agiter à température ambiante pendant 12 heures. On verse le milieu réactionnel dans l'eau glacée, extrait avec de l'éther éthylique, décante la phase organique, sèche sur sulfate de magnésium, évapore.Le résidu obtenu est chromatographié sur colonne de silice, élué avec un mélange d'hexane et de dichlorométhane (60-40). Après évaporation des solvants, on recueille 6,35 g (74%) de l'ester attendu de point de fusion 107-8°C.

### EXEMPLE 2

### Acide 2-(5,6,7,8-tétrahydro-5,5,8,8-tétraméthyl-2-anthracényl)-4-thiophène carboxylique

Dans un ballon, on introduit 6,3 g (16 mmoles) de l'ester précédent et 100 ml d'une solution de soude méthanolique 2N et chauffe à reflux pendant une heure. On évapore à sec le milieu réactionnel, reprend le résidu par l'eau, acidifie à pH 1 avec de l'acide chlorhydrique concentré et filtre le solide. On recristallise le produit obtenu dans un mélange alcool éthylique-eau et recueille 4,5 g (77%) de l'acide attendu de point de fusion 223-5°C.

### EXEMPLE 3

### 4-(5,6,7,8-tétrahydro-5,5,8,8-tétraméthyl-2-anthracényl)-2-thiophène carboxylate de méthyle

De manière analogue à l'exemple 1, à partir de 9,5 g (30 mmoles) de 5,6,7,8-tétrahydro-5,5,8,8-tétraméthyl-2-bromoanthracène et de 4,6 g (21 mmoles) de 4-bromo-2-thiophènecarboxylate de méthyle, on obtient 2,87 g (36%) de l'ester méthylique attendu sous forme d'un solide amorphe.

### EXEMPLE 4

### Acide 4-(5,6,7,8-tétrahydro-5,5,8,8-tétraméthyl-2-anthracényl)-2-thiophène carboxylique

De manière analogue à l'exemple 2, à partir de 2,8 g (7,5 mmoles) de 4-(5,6,7,8-tétrahydro-5,5,8,8-tétraméthyl-2-anthracényl)-2-thiophènecarboxylate de méthyle, on obtient 2,4 g (86%) de l'acide attendu de point de fusion 207-8°C.

### EXEMPLE 5

### 6-(5,6,7,8-tétrahydro-5,5,8,8-tétraméthyl-2-anthracényl)-nicotinate d'éthyle

De manière analogue à l'exemple 1, à partir de 8,8 g (28 mmoles) de 5,6,7,8-tétrahydro-5,5,8,8-tétraméthyl-2-bromoanthracène et de 3,7 g (20 mmoles) de 6-chloronicotinate d'éthyle, on obtient 3,9 g (51%) de l'ester éthylique attendu de point de fusion 130-2°C.

### EXEMPLE 6

### Acide 6-(5,6,7,8-tétrahydro-5,5,8,8-tétraméthyl-2-anthracényl)nicotinique

De manière analogue à l'exemple 2, à partir de 3,9 g (10 mmoles) de l'ester éthylique précédent on obtient 3,5 g (99%) de l'acide attendu de point de fusion 291-3°C.

### EXEMPLE 7

### Acide N-méthyl-4-(5,6,7,8-tétrahydro-5,5,8,8-tétraméthyl-2-anthracényl)-2-pyrrole carboxylique.

### (a) 2-trichloroacétylpyrrole.

Dans un tricol, on introduit 45 g (247 mmoles) de chlorure de trichloroacétyle et 100 ml d'éther éthylique. On ajoute goutte à goutte une solution de 15,4 g (230 mmoles) de pyrrole dans 100ml d'éther éthylique et agite à température ambiante une heure, on ajoute ensuite lentement une solution de 20 g de carbonate de potassium dans 60 ml d'eau. On décante la phase organique, sèche sur sulfate de magnésium, évapore, triture le résidu dans l'hexane et filtre. On recueille 42,7 g (87%) de produit attendu de point de fusion 78-9°C.

### (b) 4-iodo-2-trichloroacétylpyrrole.

Dans un tricol et sous courant d'azote, on introduit 8,4 g (39,5 mmoles) de 2-trichloroacétylpyrrole et 100 ml de chloroforme et ajoute sucessivement 8,8 g (39,5 mmoles) de trifluoroacétate d'argent et 10,16 g (39,5 mmoles) d'iode. On agite à température ambiante pendant une heure, verse le milieu réactionnel dans la glace, extrait avec du dichlorométhane, décante la phase organique, sèche sur sulfate de magnésium, évapore. Le résidu obtenu est trituré dans l'hexane et filtré, on recueille 8,2 g (61 %) du produit attendu de point de fusion 118-9°C.

### (c) 4-iodo-2-pyrrolecarboxylate de méthyle.

Dans un ballon, on introduit 8,2 g (24 mmoles) de 4-iodo-2-trichloroacétyl pyrrole 100 ml de méthanol et ajoute 2 g (36 mmoles) de méthylate de sodium. On agite à température ambiante pendant quatre heures, évapore à sec le milieu réactionnel,reprend le résidu obtenu par l'eau et l'éther éthylique, décante la phase organique, sèche sur sulfate de magnésium, évapore. Le résidu obtenu est trituré dans l'heptane, filtré, on recueille 4,9 g (81 %) de l'ester attendu de point de fusion 77-8°C.

### (d) N-méthyl-4-iodo-2-pyrrolecarboxylate de méthyle.

Dans un tricol on introduit 780 mg (25,9 mmoles) d'hydrure de sodium (80% dans l'huile) et 20 ml de DMF, on ajoute goutte à goutte une solution de 6,5 g (25,9 mmoles) de 4-iodo-2-pyrrolecarboxylate de méthyle dans 50 ml de DMF et agite jusqu'à cessation du dégagement gazeux. On ajoute ensuite 2,1 ml (33,6 mmoles) d'iodométhane et agite à température ambiante deux heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (40-60). On recueille 4,5 g (65%) de N-méthyl-4-iodo-2-pyrrolecarboxylate de méthyle de point de fusion 64-5°C.

### (e) acide 5,6,7,8-tétrahydro-5,5,8,8-tétraméthyl-2-anthracénylboronique.

Dans un tricol et sous courant d'azote, on introduit 5 g (15,8 mmoles) de 2-bromo-5,6,7,8-tétrahydro-5,5,8,8-tétraméthylanthracéne et 50 ml de THF. A -78°C, on ajoute goutte à goutte 7,6 ml (19 mmoles) de n-butyllithium (2,5M dans l'hexane) et agite 15', à cette même température on ajoute 4 ml (35 mmoles) de triméthylborate et agite pendant 2 heures. A -50°C on ajoute 23 ml d'acide chlorhydrique (1N) et laisse remonter à trempérature ambiante. On extrait le milieu réactionnel avec de l'éther éthylique, décante la phase organique, sèche sur sulfate de magnésium, évapore.Le résidu obtenu est trituré dans l'heptane, filtré, sèché. On recueille 2,8 g (63%) de l'acide boronique attendu de point de fusion 220-5°C.

### (f) N-méthyl-4-(5,6,7,8-tétrahydro-5,5,8,8-tétraméthyl-2-anthracényl)-2-pyrrole carboxylate de méthyle.

Dans un tricol et sous courant d'azote, on introduit 231 mg (0,2 mmole) de tétrakis(triphénylphosphine)palladium(0) 50 ml de toluène et 1,75 g (6,6 mmoles) de N-méthyl-4-iodo-2-pyrrolecarboxylate de méthyle et agite à température ambiante 20'. On ajoute ensuite 2,8 g (10 mmoles) d'acide 5,6,7,8-tétrahydro-5,5,8,8-tétraméthyl-2-anthracénylboronique 6,6 ml d'une solution aqueuse de carbonate de potassium (2N) et chauffe à reflux pendant 8 heures. On évapore à sec le milieu réactionnel, reprend par l'eau et l'éther éthylique, décante la phase organique, sèche sur sulfate de magnésium, évapore. Le résidu est purifié par chromatographie sur colonne de silice élué avec un mélange de dichlorométhane et d'hexane (80-20). On obtient 840 mg (47%) de N-méthyl-4-(5,6,7,8-tétrahydro-5,5,8,8-tétraméthyl-2-anthracényl)-2-pyrrole carboxylate de méthyle.

### (g) acide N-méthyl-4-(5,6,7,8-tétrahydro-5,5,8,8-tétraméthyl-2-anthracényl)-2-pyrrole carboxylique.

De manière analogue à l'exemple 2 à partir de 840 mg (2,2 mmoles) de N-méthyl-4-(5,6,7,8-tétrahydro-5,5,8,8-tétraméthyl-2-anthracényl)-2-pyrrole carboxylate de méthyle, on obtient 680 mg (84%) d'acide attendu de point de fusion 180-4°C.

### EXEMPLE 8

### Acide 4-(5,6,7,8-tétrahydro-5,5,8,8-tétraméthyl-2-anthracényl)-2-pyrrole carboxylique.

### (a) 4-(5,6,7,8-tétrahydro-5,5,8,8-tétraméthyl-2-anthracényl)-2-pyrrolecarboxylate de méthyle.

De manière analogue à l'exemple 7(f) par réaction de 2,1 g (7,5 mmoles) d'acide 5,6,7,8-tétrahydro-5,5,8,8-tétraméthyl-2-anthracénylboronique avec 1,25 g (5 mmoles) de 4-iodo-2-pyrrolecarboxylate de méthyle [préparé à l'exemple 7(c)], on obtient 750 mg (42%) de 4-(5,6,7,8-tétrahydro-5,5,8,8-tétraméthyl-2-anthracényl)-2-pyrrole carboxylate de méthyle de point de fusion 140-3°C.

### (b) acide 4-(5,6,7,8-tétrahydro-5,5,8,8-tétraméthyl-2-anthracényl)-2-pyrrole carboxylique.

De manière analogue à l'exemple 2 à partir de 750 mg (2 mmoles) de 4-(5,6,7, 8-tétrahydro-5,5,8,8-tétraméthyl-2-anthracényl)-2-pyrrolecarboxylate de méthyle, on obtient 180 mg (25%) de l'acide attendu de point de fusion 234-8°C.

### EXEMPLE 9

### 2-hydroxy-4-(5,6,7,8-tétrahydro-5,5,8,8-tétraméthyl-2-anthracényl)benzoate de méthyle.

De manière analogue à l'exemple 7(f) par réaction de 8,5 g (30,3 mmoles) d'acide 5,6,7,8-tétrahydro-5,5,8,8-tétraméthyl-2-anthracénylboronique avec 5,6 g (20 mmoles) de 2-hydroxy-4-iodobenzoate de méthyle, on obtient 5,4 g (69%) de 2-hydroxy-4-(5,6,7,8-tétrahydro-5,5,8,8-tétraméthyl-2-anthracényl)benzoate de méthyle de point de fusion 145-6°C.

### EXEMPLE 10

### Acide 2-hydroxy-4-(5,6,7,8-tétrahydro-5,5,8,8-tétraméthyl-2-anthracényl) benzoïque.

De manière analogue à l'exemple 2 à partir de 900 mg (2,3 mmoles) de 2-hydroxy4-(5,6,7,8-tétrahydro-5,5,8,8-tétraméthyl-2-anthracényl)benzoate de méthyle, on obtient 460 mg (53%) d'acide attendu de point de fusion 249-52°C.

### EXEMPLE 11

### Acide 2-méthoxy-4-(5,6,7,8-tétrahydro-5,5,8,8-tétraméthyl-2-anthracényl)benzoïque.

### (a) 2-méthoxy-4-(5,6,7,8-tétrahydro-5,5,8,8-tétraméthyl-2-anthracényl) benzoate de méthyle.

Dans un tricol on introduit 130 mg (4,2 mmoles) d'hydrure de sodium (80% dans l'huile) et 20 ml de DMF, on ajoute goutte à goutte une solution de 1,6 g (4,2 mmoles) de 2-hydroxy-4-(5,6,7,8-tétrahydro-5,5,8,8-tétraméthyl-2-anthracényl) benzoate de méthyle dans 50 ml de DMF et agite jusqu'à cessation du dégagement gazeux. On ajoute ensuite 340 µl (5,5 mmoles) d'iodométhane et agite à température ambiante deux heures. On verse le milieu réactionnel dans l'eau, extrait avec de l'éther éthylique, décante la phase organique, sèche sur sulfate de magnésium, évapore. On recueille 1,6 g (96%) du produit attendu sous forme d'une huile incolore.

### (b) acide 2-méthoxy-4-(5,6,7,8-tétrahydro-5,5,8,8-tétraméthyl-2-anthracényl) benzoïque.

De manière analogue à l'exemple 2 à partir de 1,6 g (4 mmoles) de 2-méthoxy-4-(5,6,7,8-tétrahydro-5,5,8,8-tétraméthyl-2-anthracényl) benzoate de méthyle, on obtient 1,2 g (78%) de l'acide attendu de point de fusion 177-8°C.

### EXEMPLE 12

### Acide 2-hydroxy-4-[7-(1-adamantyl)-6-benzyloxy-2-naphtyl]benzoïque.

### (a) 7-(1-adamantyl)-6-benzyloxy-2-bromonaphtalène.

De manière analogue à l'exemple 11 par réaction de 12,5 g (35 mmoles) de 7-(1-adamantyl)-6-hydroxy-2-bromonaphtalène avec 5 ml (42 mmoles) de bromure de benzyle, on obtient 12,5 g (80%) du produit attendu de point de fusion 150-1°C.

### (b) acide 7-(1-adamantyl)-6-benzyloxy-2-naphtylboronique.

De manière analogue à l'exemple 7(e) à partir de 3 g (6,7 mmoles) de 7-(1-adamantyl)-6-benzyloxy-2-bromonaphtalène, on obtient 2,8 g (100%) d'acide boronique attendu qui est utilisé telquel pour la suite de la synthèse.

### (c) 2-hydroxy-4-[7-(1-adamantyl)-6-benzyloxy-2-naphtyl]benzoate de méthyle.

De manière analogue à l'exemple 7(f) par réaction de 5,52 g (13,4 mmoles) d'acide 7-(1-adamantyl)-6-benzyloxy-2-naphtylboronique avec 2,46 g (8,8 mmoles) de 2-hydroxy-4-iodobenzoate de méthyle, on obtient 1,65 g (36%) du produit attendu.

### (d) acide 2-hydroxy-4-[7-(1-adamantyl)-6-benzyloxy-2-naphtyl]benzoïque.

De manière analogue à l'exemple 2 à partir de 930 mg (1,8 mmole) de 2-hydroxy-4-[7-(1-adamantyl)-6-benzyloxy-2-naphtyl]benzoate de méthyle, on obtient 710 mg (79%) de l'acide attendu de point de fusion 263-4°C.

### EXEMPLE 13

### Acide 2-hydroxy-4-[7-(1-adamantyl)-6-hydroxy-2-naphtyl]benzoïque.

### (a) 2-hydroxy-4-(7-(1-adamantyl)-6-hydroxy-2-naphtyl]benzoate de méthyle.

Dans un réacteur on introduit 1,5 g (2,9 mmoles) de 2-hydroxy-4-[7-(1-adamantyl)-6-benzyloxy-2-naphtyl]benzoate de méthyle, 450 mg de palladium sur charbon (10%) et 50 ml de dioxanne. On ajoute 5 gouttes d'acide acétique et hydrogène à 50°C et sous une pression de 6,5 bars d'hydrogène pendant 4 heures. On filtre le catalyseur, lave avec deux fois 20 ml de dioxanne, évapore les filtrats. Le résidu obtenu est purifié par chromatographie sur colonne de silice élué avec un mélange de dichlorométhane et d'hexane (50-50). On recueille 830 mg (67%) du produit attendu.

### (b) acide 2-hydroxy-4-[7-(1-adamantyl)-6-hydroxy-2-naphtyl]benzoïque.

De manière analogue à l'exemple 2 à partir de 400 mg (0,9 mmole) de 2-hydroxy-4-[7-(1-adamantyl)-6-hydroxy-2-naphtyl]benzoate de méthyle, on obtient 290 mg (75%) de l'acide attendu de point de fusion 261-4°C.

### EXEMPLE 14

### Acide 2-hydroxy-4-[7-(1-adamantyl)-6-hexyloxy-2-naphtyl]benzoïque.

### (a) 2-hydroxy-4-[7-(1-adamantyl)-6-hexyloxy-2-naphtyl]benzoate de méthyle.

De manière analogue à l'exemple 11 par réaction de 430 mg (1 mmole) de 2-hydroxy-4-[7-(1-adamantyl)-6-hydroxy-2-naphtyl]benzoate de méthyle avec 180 µl (1,2 mmoles) de 6-iodohexane, on obtient 280 mg (55%) de 2-hydroxy-4-[7-(1-adamantyl)-6-hexyloxy-2-naphtyl]benzoate de méthyle.

### (b) acide 2-hydroxy-4-[7-(1-adamantyl)-6-hexyloxy-2-naphtyl]benzoïque.

De manière analogue à l'exemple 2 à partir de 100 mg (0,2 mmole) de 2-hydroxy-4-[7-(1-adamantyl)-6-hexyloxy-2-naphtyl]benzoate de méthyle, on obtient 90 mg (92%) de l'acide attendu de point de fusion 281-3°C.

### EXEMPLE 15

### Acide 4-[7-(1-adamantyl)-6-méthoxyéthoxyméthoxy-2-naphtyl]benzoïque.

### (a) 4-[7-(1-adamantyl)-6-benzyloxy-2-naphtyl]benzoate de méthyle.

De manière analogue à l'exemple 7(f) par réaction de 2,8 g (6,7 mmoles) d'acide 7-(1-adamantyl)-6-benzyloxy-2-naphtylboronique avec 950 mg (4,4 mmoles) de 4-bromobenzoate de méthyle, on obtient 1,6 g (72%) du produit attendu.

### (b) 4-[7-(1-adamantyl)-6-hydroxy-2-naphtyl]benzoate de méthyle.

De manière analogue à l'exemple 13(a) à partir de 1,38 g (2,75 mmoles) de 4-[7-(1-adamantyl)-6-benzyloxy-2-naphtyl]benzoate de méthyle, on obtient 980 mg (86%) de 4-[7-(1-adamantyl)-6-hydroxy-2-naphtyl]benzoate de méthyle sous forme d'une huile.

### (c) 4-[7-(1-adamantyl)-6-méthoxyéthoxyméthoxy-2-naphtyl]benzoate de méthyle.

De maniére analogue à l'exemple 12 par réaction de 980 mg (2,4 mmoles) de 4-[7-(1-adamantyl)-6-hydroxy-2-naphtyl]benzoate de méthyle avec 330 µl (28,6 mmoles) de chlorure de méthoxyéthoxyméthane, on obtient 650 mg (55%) du produit attendu sous forme d'une huile.

### (d) acide 4-[7-(1-adamantyl)-6-méthoxyéthoxyméthoxy-2-naphtyl]benzoïque.

De manière analogue à l'exemple 2 à partir de 650 mg (1,3 mmole) de 4-[7-(1-adamantyl)-6-méthoxyéthoxyméthoxy-2-naphtyl]benzoate de méthyle, on obtient 580 mg (92%) de l'acide attendu de point de fusion 234-6°C.

### EXEMPLE 16

### Acide 3-méthyl-4-(5,6,7,8-tétrahydro-5,5,8,8-tétraméthyl-2-anthracényl)benzoïque.

### (a) acide 3-méthyl-4-iodobenzoïque.

Dans un tricol on introduit 20 g (0,132 mole) d'acide 3-méthyl-4-amino benzoïque 175 ml d'acide sulfurique (20%). A -10°C on ajoute goutte à goutte une solution de 11,9 g (0,172 mole) de nitrite de sodium dans 50 ml d'eau et agite pendant deux heures. Cette solution est introduite goutte à goutte par l'intermédiaire d'une ampoule réfrigérée à -5°C dans une solution de 35 g (0,211 mole) d'iodure de potassium 35,2 g (0,185 mole) d'iodure de cuivre et 175 ml d'acide sulfurique (20%). On agite pendant huit heures, filtre le milieu réactionnel, dissout le solide obtenu dans l'acétate d'éthyle, lave à l'eau puis avec une solution de sulfite de sodium,sèche sur sulfate de magnésium, évapore. On recueille 24,4 g (70%) d'acide 3-méthyl-4-iodobenzoïque de point de fusion 205-10°C.

### (b) 3-méthyl-4-iodobenzoate de méthyle.

Dans un ballon on introduit 24,4 g (0,093 mole) d'acide 3-méthyl-4-iodobenzoïque 250 ml de méthanol et ajoute goutte à goutte 2,5 ml d'acide sulfurique concentré. On chauffe à reflux pendant douze heures, évapore le milieu réactionnel, reprend avec acétate d'éthyle et eau, décante la phase organique, sèche sur sulfate de magnésium, évapore. Le résidu est trituré dans le méthanol, filtré, on recueille 21,9 g (85%) de l'ester méthylique attendu de point de fusion 58-9°C.

### (c) 3-méthyl-4-(5,6,7,8-tétrahydro-5,5,8,8-tétraméthyl-2-anthracényl)benzoate de méthyle.

De manière analogue à l'exemple 7(f) par réaction de 2,8 g (10 mmoles) d'acide 5,6,7,8-tétrahydro-5,5,8,8-tétraméthyl-2-anthracénylboronique avec 1,84 g (6,7 mmoles) de 3-méthyl-4-iodobenzoate de méthyle, on obtient 1,37 g (53%) de 3-méthyl-4-(5,6,7,8-tétrahydro-5,5,8,8-tétraméthyl-2-anthracényl) benzoate de méthyle sous forme d'une huile jaune.

### (d) acide 3-méthyl-4-(5,6,7,8-tétrahydro-5,5,8,8-tétraméthyl-2-anthracényl) benzoïque.

De manière analogue à l'exemple 2 à partir de 860 mg (2,2 mmoles) de 3-méthyl-4-(5,6,7,8-tétrahydro-5,5,8,8-tétraméthyl-2-anthracényl) benzoate de méthyle, on obtient 770 mg (93%) d'acide attendu de point de fusion 248-50°C.

### EXEMPLE 17

### Acide N-propyl-4-(5,6,7,8-tétrahydro-5,5,8,8-tétraméthyl-2-anthracényl)-2-pyrrole carboxylique.

### (a) N-propyl-4-iodo-2-pyrrolecarboxylate de méthyle.

De manière analogue à l'exemple 7(d) par réaction de 1,96 g (7,8 mmoles) de 4-iodo-2-pyrrolecarboxylate de méthyle avec 940 µl (9,6 mmoles) de 3-iodopropane, on obtient 1,48 g (64%) de N-propyl-4-iodo-2-pyrrolecarboxylate de méthyle sous forme d'une huile légèremént jaune.

### (b) N-propyl-4-(5,6,7,8-tétrahydro-5,5,8,8-tétraméthyl-2-anthracényl)-2-pyrrole carboxylate de méthyle.

De manière analogue à l'exemple 7(f) par réaction de 1,7 g (6 mmole) d'acide 5,6,7,8-tétrahydro-5,5,8,8-tétraméthyl-2-anthracénylboronique avec 1,47 g (5 mmoles) de N-propyl-4-iodo-2-pyrrolecarboxylate de méthyle, on obtient 450 mg (22%) de N-propyl-4-(5,6,7,8-tétrahydro-5,5,8,8-tétraméthyl-2-anthracényl)-2-pyrrole carboxylate de méthyle.

### (c) acide N-propyl-4-(5,6,7,8-tétrahydro-5,5,8,8-tétraméthyl-2-anthracényl)-2-pyrrole carboxylique.

De manière analogue à l'exemple 2 à partir de 450 mg (1,12 mmole) de N-propyl-4-(5,6,7,8-tétrahydro-5,5,8,8-tétraméthyl-2-anthracényl)-2-pyrrole carboxylate de méthyle, on obtient 230 mg (54%) d'acide attendu de point de fusion 143-5°C.

### EXEMPLE 18

### Acide 2-propyloxy-4-(5,6,7,8-tétrahydro-5,5,8,8-tétraméthyl-2-anthracényl)benzoïque.

### (a) 2-propyloxy-4-(5,6,7,8-tétrahydro-5,5,8,8-tétraméthyl-2-anthracényl) benzoate de méthyle.

De manière analogue à l'exemple 12 par réaction de 2 g (5,1 mmoles) de 2-hydroxy-4-(5,6,7,8-tétrahydro-5,5,8,8-tétraméthyl-2-anthracényl)benzoate de méthyle avec 600 µl (6,1 mmoles) de 3-odopropane, on obtient 1,16 g (54%) de 2-propyloxy-4-(5,6,7,8-tétrahydro-5,5,8,8-tétraméthyl-2-anthracényl) benzoate de méthyle.

### (b) acide 2-propyloxy-4-(5,6,7,8-tétrahydro-5,5,8,8-tétraméthyl-2-anthracényl)benzoïque.

De manière analogue à l'exemple 2 à partir de 1,15 g (2,75 mmoles) de 2-propyloxy4-(5,6,7,8-tétrahydro-5,5,8,8-tétraméthyl-2-anthracényl) benzoate de méthyle, on obtient 760 mg (68%) d'acide attendu de point de fusion 137-8°C.

### EXEMPLE 19

### Acide 2-hexyloxy-4-(5,6,7,8-tétrahydro-5,5,8,8-tétraméthyl-2-anthracényl)benzoïque.

### (a) 2-hexyloxy-4-(5,6,7,8-tétrahydro-5,5,8,8-tétraméthyl-2-anthracényl)benzoate de méthyle.

De manière analogue à l'exemple 12 par réaction de 2 g (5,1 mmoles) de 2-hydroxy-4-(5,6,7,8-tétrahydro-5,5,8,8-tétraméthyl-2-anthracényl)benzoate de méthyl avec 1,1 ml (6,1 mmoles) de 6-iodohexane, on obtient 1,67 g (64%) de 2-hexyloxy-4-(5,6,7,8-tétrahydro-5,5,8,8-tétraméthyl-2-anthracényl) benzoate de méthyle de point de fusion 102-4°C.

### (b) acide 2-hexyloxy-4-(5,6,7,8-tétrahydro-5,5,8,8-tétraméthyl-2-anthracényl)benzoïque.

De manière analogue à l'exemple 2 à partir de 1,67 g (3,5 mmoles) de 2-hexyloxy-4-(5,6,7,8-tétrahydro-5,5,8,8-tétraméthyl-2-anthracényl) benzoate de méthyle, on obtient 1,35 g (83%) d'acide attendu de point de fusion 105-6°C.

### EXEMPLE 20

### Acide 5-[7-(1-adamantyl)-6-benzyloxy-2-naphtyl]-2-thiophènecarboxylique.

### (a) 5-[7-(1-adamantyl)-6-benzyloxy-2-naphtyl]-2-thiophénecarboxylate de méthyle.

De manière analogue à l'exemple 7(f) par réaction de 1,5 g (3,6 mmoles) d'acide 7-(1-adamantyl)-6-benzyloxy-2-naphtylboronique avec 400 mg (1,8 mmole) de 5-bromo-2-thiophènecarboxylate de méthyle, on obtient 600 mg (65%) du produit attendu de point de fusion 170-1°C.

### (b) acide 5-[7-(1-adamantyl)-6-benzyloxy-2-naphtyl]-2-thiophènecarboxylique.

De manière analogue à l'exemple 2 à partir de 600 mg (1,2 mmole) de 5-[7-(1-adamantyl)-6-benzyloxy-2-naphtyl]-2-thiophènecarboxylate de méthyle, on obtient 460 mg (79%) d'acide attendu de point de fusion 271-3°C.

### EXEMPLE 21

### Acide 4-[7-(1-adamantyl)-6-benzyloxy-2-naphtyl]benzoïque.

### (a) 4-[7-(1-adamantyl)-6-benzyloxy-2-naphtyl]benzoate de méthyle.

De manière analogue à l'exemple 7(f) par réaction de 1,5 g (3,6 mmoles) d'acide 7-(1-adamantyl)-6-benzyloxy-2-naphtylboronique avec 500 mg (1,9 mmole) de 4-iodobenzoate de méthyle, on obtient 320 mg (33%) du produit attendu de point de fusion 170-3°C.

### (b) acide 4-[7-(1-adamantyl)-6-benzyloxy-2-naphtyl]benzoïque.

De manière analogue à l'exemple 2 à partir de 320 mg (0,6 mmole) de 4-[7-(1-adamantyl)-6-benzyloxy-2-naphtyl]benzoate de méthyle, on obtient 195 mg (63%) d'acide attendu de point de fusion 305-10°C.

### EXEMPLE 22

### Acide 2-chloro-4-(5,6,7,8-tétrahydro-5,5,8,8-tétraméthyl-2-anthracényl)benzoïque.

### (a) acide 2-chloro-4-iodobenzoïque.

De manière analogue à l'exemple 16(a) à partir de 10 g (58,3 mmoles) d'acide 2-chloro-4-aminobenzoïque, on recueille 14,26 g (86%) d'acide 2-chloro-4-iodobenzoïque.

### (b) 2-chloro-4-iodobenzoate de méthyle.

De manière analogue à l'exemple 16(b) à partir de 13,9 g (49,2 mmoles) d'acide 2-chloro-4-iodobenzoïque on obtient 11,52 g (79%) de l'ester méthylique attendu sous forme d'une huile.

### (c) 2-chloro-4-(5,6,7,8-tétrahydro-5,5,8,8-tétraméthyl-2-anthracényl) benzoate de méthyle.

De manière analogue à l'exemple 7 (f) par réaction de 2,8 g (9,9 mmoles) d'acide 5,6,7,8-tétrahydro-5,5,8,8-tétraméthyl-2-anthracénylboronique avec 2,5 g (8,27 mmoles) de 2-chloro-4-iodobenzoate de méthyle, on obtient 1,8 g (67%) de l'ester méthylique attendu.

### (d) acide 2-chloro-4-(5,6,7,8-tétrahydro-5,5,8,8-tétraméthyl-2-anthracényl) benzoïque.

De manière analogue à l'exemple 2 à partir de 2,2 g (5,4 mmoles) de 2-chloro-4-(5,6,7,8-tétrahydro-5,5,8,8-tétraméthyl-2-anthracényl) benzoate de méthyle, on obtient 2,1 g (99%) de l'acide attendu de point de fusion 213-5°C.

### EXEMPLE 23

### Acide 2-hydroxy-4-[7-(1-adamantyl)-6-(4-fluorobenzyl)oxy-2-naphtyl]benzoïque.

### (a) 7-(1-adamantyl)-6-(4-fluorobenzyl)oxy-2-bromonaphtalène.

De manière analogue à l'exemple 11 par réaction de 1,1 g (3 mmoles) de 7-(1-adamantyl)-6-hydroxy-2-bromonaphtalène avec 420 µl (3,3 mmoles) de bromure de 4-fluorobenzyle, on obtient 1,2 g (86%) du produit attendu sous forme d'une huile incolore.

### (b) acide 7-(1-adamantyl)-6-(4-fluorobenzyl)oxy-2-naphtylboronique.

De manière analogue à l'exemple 7(e) à partir de 1,14 g (2,45 mmoles) de 7-(1-adamantyl)-6-(4-fluorobenzyl)oxy-2-bromonaphtalène, on obtient 560 mg (57%) d'acide boronique attendu.

### (c) 2-hydroxy-4-[7-(1-adamantyl)-6-(4-fluorobenzyl)oxy-2-naphtyl]benzoate de méthyle.

De manière analogue à l'exemple 7(f) par réaction de 560 mg (1,41 mmole) d'acide 7-(1-adamantyl)-6-(4-fluorobenzyl)oxy-2-naphtylboronique avec 330 mg (1,17 mmole) de 2-hydroxy-4-iodobenzoate de méthyle, on obtient 490 mg (78%) de l'ester attendu de point de fusion 189-91°C.

### (d) acide 2-hydroxy-4-(7-(1-adamantyl)-6-(4-fluorobenzyl)oxy-2-naphtyl]benzoïque.

De manière analogue à l'exemple 2 à partir de 490 mg (0,91 mmole) de 2-hydroxy-4-[7-(1-adamantyl)-6-(4-fluorobenzyl)oxy-2-naphtyl]benzoate de méthyle, on obtient 440 mg (92%) d'acide attendu de point de fusion 240-1°C.

### EXEMPLE 24

### Acide 2-[7-(1-adamantyl)-6-hydroxy-2-naphtyl]-4-thiophènecarboxylique.

### (a) 7-(1-adamantyl)-6-tert-butyldiméthylsilyloxy-2-bromonaphtalène.

Dans un tricol on introduit successivement 11,9 g (33,3 mmoles) de 7-(1-adamantyl)-6-hydroxy-2-bromonaphtalène 120 ml de DMF 5,1 ml (36,6 mmoles) de triéthylamine et 203 mg de 4-diméthylaminopyridine. On ajoute goutte à goutte une solution de 5,52 g (36,6 mmoles) de chlorure de tert-butyldiméthylsilane et agite à température ambiante pendant 12 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec de l'heptane, on recueille 12 g (76%) de 7-(1-adamantyl)-6-tert-butyldiméthylsilyloxy-2-bromonaphtalène de point de fusion 120-1°C.

### (b) acide 7-(1-adamantyl)-6-tert-butyldiméthylsilyloxy-2-naphtylboronique.

De manière analogue à l'exemple 7(e) à partir de 11,9 g (25,4 mmoles) de 7-(1-adamantyl)-6-tert-butyldiméthylsilyloxy-2-bromonaphtalène, on obtient 8,37 g (75%) d'acide boronique attendu de point de fusion 221-2°C.

### (c) 2-[7-(1-adamantyl)-6-tert-butyldiméthylsilyloxy-2-naphtyl]-4-thiophènecarboxylate d'éthyle.

De manière analogue à l'exemple 7(f) par réaction de 8,37 g (19,2 mmoles) d'acide 7-(1-adamantyl)-6-tert-butyldiméthylsilyloxy-2-naphtylboronique avec 4,45 g (18,9 mmoles) de 2-bromo-4-thiophènecarboxylate d'éthyle, on obtient 8,87 g (86%) de l'ester éthylique attendu de point de fusion 75-6°C.

### (d) Acide 2-[7-(1-adamantyl)-6-hydroxy-2-naphtyl]-4-thiophènecarboxylique.

De manière analogue à l'exemple 2 à partir de 550 mg (1 mmole) de 2-[7-(1-adamantyl)-6-tert-butyldiméthylsilyloxy-2-naphtyl]-4-thiophènecarboxylate d'éthyle, on obtient 186 mg (46%) de l'acide attendu de point de fusion 312-4°C.

### EXEMPLE 25

### Acide 2-[7-(1-adamantyl)-6-méthoxy-2-naphtyl]-4-thiophènecarboxylique.

### (a) 2-[7-(1-adamantyl)-6-hydroxy-2-naphtyl]-4-thiophènecarboxylate d'éthyle.

Dans un ballon, on introduit 8,29 g (15,2 mmoles) de 2-[7-(1-adamantyl)-6-tert-butyldiméthylsilyloxy-2-naphtyl]-4-thiophènecarboxylate d'éthyle et 60 ml de THF. On ajoute goutte à goutte une solution de 15,2 ml (16,6 mmoles) de fluorure de tétrabutylammonium dans le THF (1.1 N) et agite à température ambiante pendant 2 heures. On verse le milieu réactionnel dans l'eau, extrait avec de l'éther éthylique. décante la phase organique, sèche sur sulfate de magnésium, évapore. Le résidu obtenu est trituré dans l'heptane, filtré, séché. On recueille 6.12 g (93%) de 2-[7-(1-adamantyl)-6-hydroxy-2-naphtyl]-4-thiophènecarboxylate d'éthyle de point de fusion 199-200°C.

### (b) 2-[7-(1-adamantyl)-6-méthoxy-2-naphtyl]-4-thiophènecarboxylate d'éthyle.

De manière analogue à l'exemple 11 par réaction de 1 g (2,31 mmoles) de 2-[7-(1-adamantyl)-6-hydroxy-2-naphtyl]-4-thiophènecarboxylate d'éthyle avec 158 µl (2,54 mmoles) d'iodométhane, on obtient 632 mg (61%) du produit attendu de point de fusion 159-160°C.

### (c) acide 2-[7-(1-adamanty)-6-méthoxy-2-naphtyl]-4-thiophènecarboxylique

De manière analogue à l'exemple 2 à partir de 625 mg (1,4 mmole) de 2-[7-(1-adamantyl)-6-méthoxy-2-naphtyl]-4-thiophènecarboxylate d'éthyle, on obtient 469 mg (80%) d'acide attendu de point de fusion 314-6°C.

### EXEMPLE 26

### Acide 2-[7-(1-adamantyl)-6-benzyloxycarbonyl-2-naphtyl]-4-thiophènecarboxylique

### (a) 2-[7-(1-adamantyl)-6-trifluorométhylsulfonyloxy-2-naphtyl]-4-thiophène carboxylate d'éthyle.

A une solution refroidit à-78°C de 5,11 g (11,8 mmoles) de 2-[7-(1-adamantyl)-6-hydroxy-2-naphtyl]-4-thiophènecarboxylate d'éthyle 2,85 ml (35,4 mmoles) de pyridine 14 mg de 4-diméthylaminopyridine dans 100 ml de dichlorométhane. on ajoute goutte à goutte 2,4 ml (14,2 mmoles) d'anhydride trifluorométhanesulfonique et agite à température ambiante pendant 12 heures. On verse le milieu réactionnel dans l'eau glacée, extrait avec de l'éther éthylique. décante la phase organique, lave avec une solution saturée de chlorure de sodium, sèche sur sulfate de magnésium, évapore. Le résidu obtenu est purifié par chromatographie sur colonne de silice élué avec un mélange de dichlorométhane et d'heptane (40-60). On recueille 1,55 g (26%) de 4-[7-(1-adamantyl)-6-trifluorométhylsulfonyloxy-2-naphtyl]-4-thiophènecarboxylate d'éthyle de point de fusion 133-4°C.

### (b) 2-[7-(1-adamantyl)-6-benzyloxycarbonyl-2-naphty]-4-thiopnènecarboxylate d'éthyle.

Dans un réacteur on introduit successivement 1.54 g (3 mmoles) de 4-[7-(1-adamantyl)-6-trifluorométhylsulfonyloxy-2-naphtyl]-4-thiophènecarboxylate d'éthyle 845 µl (6 mmoles) de triéthylamine 34 mg (5% molaire) d'acétate de palladium 168 mg (0.3 mmole) de diphénylphosphineferrocéne 3.15 ml (30 mmoles) d'alcool benzylique et 50 ml de DMF.On chauffe à 70°C et soumet à une pression de 2,5 bars de monoxyde de carbone pendant 3 heures. On verse le milieu réactionnel dans une solution aqueuse saturée en chlorure de sodium, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (10-90). On recueille 472 mg (28%) de 2-[7-(1-adamantyl)-6-benzyloxycarbonyl-2-naphtyl]-4-thiophènecarboxylate d'éthyle de point de fusion 98-100°C.

### (c) acide 2-[7-(1-adamantyl)-6-benzyloxycarbonyl-2-naphtyl]-4-thiophènecarboxylique.

De manière analogue à l'exemple 2 à partir de 462 mg (0,84 mmole) de 2-[7-(1-adamantyl)-6-benzyloxycarbonyl-2-naphtyl]-4-thiophènecarboxylate d'éthyle, on obtient 419 mg (95%) de l'acide attendu de point de fusion 205-7°C.

### EXEMPLE 27

### 4-[7-(1-adamantyl)-6-benzyloxycarbonyl-2-naphtyl]benzoate de méthyle.

### (a) 4-[7-(1-adamantyl)-6-trifluorométhylsulfonyloxy-2-naphtyl]benzoate de méthyle.

De manière analogue à l'exemple 26(a) par réaction de 5,5 g (13,3 mmoles) de 4-[7-(1-adamantyl)-6-hydroxy-2-naphtyl]benzoate de méthyle (préparation décrite dans EP 0 210 929) avec 2,7 ml (16 mmoles) d'anhydride trifluorométhane sulfonique, on obtient 1,94 g (27%) de 4-[7-(1-adamantyl)-6-trifluorométhylsulfonyl oxy-2-naphtyl]benzoate de méthyle de point de fusion 226-7°C.

### (b) 4-[7-(1-adamantyl)-6-benzyloxycarbonyl-2-naphtyl]benzoate de méthyle.

De maniére analogue à l'exemple 26(b) à partir de 1,91 g (3,5 mmoles) de 4-[7-(1-adamantyl)-6-trifluorométhylsulfonyloxy-2-naphtyl]benzoate de méthyle, on obtient 720 mg (40%) de 4-[7-(1-adamantyl)-6-benzyloxycarbonyl-2-naphtyl] benzoate de méthyle de point de fusion 143-4°C.

### EXEMPLE 28

Dans cet exemple, on a illustré diverses formulations concrètes à base des composés selon l'invention.

### A- VOIE ORALE

| (a) Comprimé de 0,2 g | |
|---|---|
| - Composé préparé à l'exemple 6 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |

| (b) Suspension buvable en ampoules de 5 ml | |
|---|---|
| - Composé préparé à l'exemple 5 | 0,001 g |
| - Glycérine | 0,500 g |
| - Sorbitol à 70% | 0,500 g |
| - Saccharinate de sodium | 0,010 g |
| - Parahydroxybenzoate de méthyle | 0,040 g |
| - Arome qs | |
| - Eau purifiée qsp | 5 ml |

| (c) Comprimé de 0,8 g | |
|---|---|
| - Composé de l'exemple 2 | 0,500 g |
| - Amidon prégélatinisé | 0,100 g |
| - Cellulose microcristalline | 0,115 g |
| - Lactose | 0,075 g |
| - Stéarate de magnésium | 0,010 g |

| (d) Suspension buvable en ampoules de 10 ml | |
|---|---|
| - Composé de l'exemple 4 | 0,200 g |
| - Glycérine | 1,000g |
| - Sorbitol à 70% | 1,000g |
| - Saccharinate de sodium | 0,010 g |
| - Parahydroxybenzoate de méthyle | 0,080 g |
| - Arome qs | |
| - Eau purifiée qsp | 10 ml |

### B- VOIE TOPIQUE

| (a) Onguent | |
|---|---|
| - Composé de l'exemple 6 | 0,020 g |
| - Myristate d'isopropyle | 81,700 g |
| - Huile de vaseline fluide | 9,100 g |
| - Silice ("Aérosil 200" vendue par DEGUSSA) | 9,180 g |

| (b) Onguent | |
|---|---|
| - Composé de l'exemple 2 | 0,300 g |
| - Vaseline blanche codex | 100 g |

| (c) Crème Eau-dans-Huile non ionique | |
|---|---|
| - Acide 6-[7-tert-butyl-6-(2-hydroxypropyl)-2-naphtyl]nicotinique | 0,100g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |

| (d) Lotion | |
|---|---|
| - Acide 6-(7-tertbutyl-6-méthoxycarbonylméthyloxy-2-naphtyl)nicotinique | 0,100g |
| - Polyéthylène glycol (PEG 400) | 69,900 g |
| - Ethanol à 95% | 30,000 g |

| (e) Onguent hydrophobe | |
|---|---|
| - Acide 6-(7-tertbutyl-6-carboxyméthyloxy-2-naphtyl) nicotinique | 0,300 g |
| - Mirystate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) | 100g |

| (f) Crème Huile-dans-Eau non ionique | |
|---|---|
| - Composé de l'exemple 5 | 1,000 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile | 100 g |

## Revendications

1. Composés aromatiques polycycliques, caractérisés par le fait qu'ils répondent à la formule générale (I) suivante : 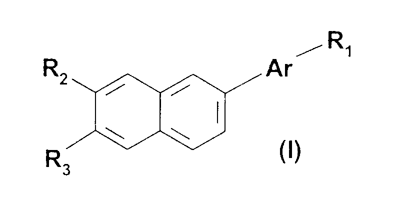 dans laquelle :
* R₁ représente :
(i) un atome d'hydrogène
(ii) un radical -CH₃
(iii) un radical -CH₂OH
(iv) un radical -O-R₄
(v) un radical -S(O)t-R₅
(vi) un radical -CO-R₆
R₄, R₅, R₆ et t ayant la signification donnée ci-après,
* Ar représente un radical choisi parmi les radicaux de formules (a)-(i) suivantes : 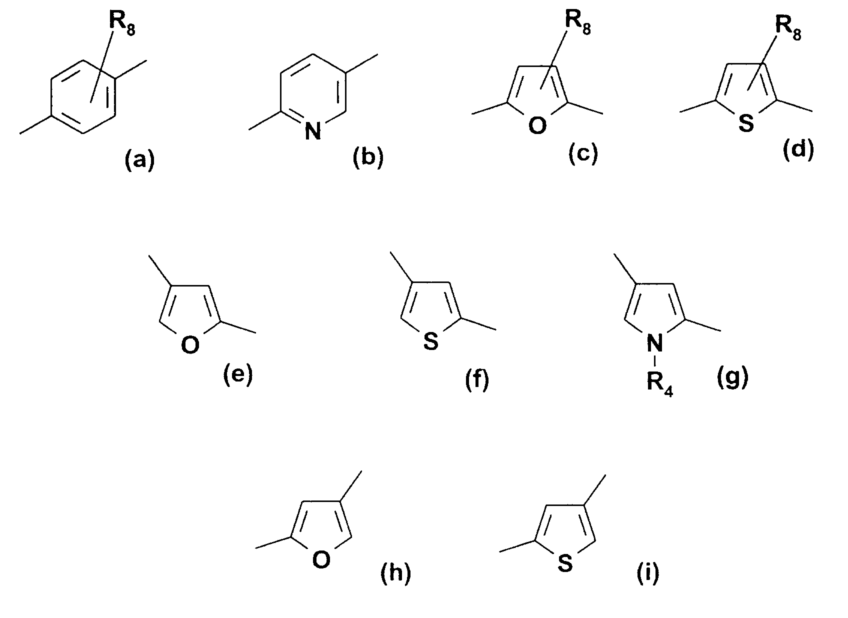 R₄ et R₈ ayant la signification donnée ci-après,
* R₂ représente un radical alkyle linéaire ou ramifié ayant de 1 à 20 atomes de carbone ou un radical cycloaliphatique,
* R₃ représente :
(a) un radical -X-(CH₂)ₘ-R₁₀
(b) un radical -(CH₂)ₘ-R₁₁
(c) un radical -CH=CH-(CH₂)ₙ-R₁₁
(d) un radical -O-CH₂-O-CH₂-CH₂-O-CH₃
X, R₁₀, R₁₁, n et m ayant la signification donnée ci-après,
étant entendu que dans tout ce qui précède :
- R₂ et R₃, pris ensemble, peuvent former avec le noyau naphtalénique adjacent un cycle à 5 ou 6 chainons éventuellement substitué par des groupes méthyle et/ou éventuellement interrompu par un atome d'oxygène ou par un radical -S(O)z-, z ayant la signification donnée ci-après,
- R₄ représente un atome d'hydrogène, un radical alkyle ayant de 1 à 6 atomes de carbone ou un radical -(CH2)p-(CO)q-R₅, p, q et R5 ayant la signification donnée ci-après,
- R₅ représente un radical alkyle inférieur ou un hétèrocycle,
- R₆ représente :
(a) un atome d'hydrogène
(b) un radical alkyle ayant de 1 à 6 atomes de carbone
(c) un radical de formule : 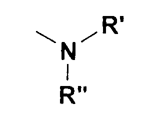 R' et R'' ayant la signification donnée ci-après,
(d) un radical -O-R₇, R₇ ayant la signification donnée ci-après,
- R₇ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre ou un reste d'amino acide ou de peptide,
- R₈ représente un atome d'halogène, un radical alkyle ayant de 1 à 6 atomes de carbone, un radical hydroxy, un radical -OR₉ ou -O-COR₉, R₉ ayant la signification donnée ci-après, ou encore un atome d'hydrogène lorsque R₂ est le radical 1-adamantyl et R₃ est différent du radical -OCH₃ ou du radical -OH,
- R₉ représente un radical alkyle ayant de 1 à 6 atomes de carbone,
- R₁₀ représente un atome d'hydrogène, un radical alkyle ayant de 1 à 6 atomes de carbone, un radical aryle, un radical aralkyle, un radical monohydroxyalkyle, un radical polyhydroxyalkyle, un radical -CO-R₆ ou bien encore, mais seulement dans le cas où m est supérieur ou égal à 2, un radical de formule : 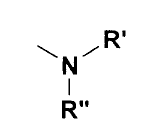 R' et R'' ayant la signification donnée ci-après,
- R₁₁ représente un atome d'hydrogène, un radical monohydroxyalkyle, un radical polyhydroxyalkyle, un radical -CO-R₆, un radical de formule : 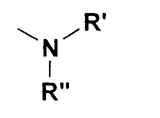 R' et R'' ayant la signification donnée ci-après,
ou bien encore, mais seulement dans le cas où m est supérieur ou égal à 1, un radical hydroxy, un radical -OR₉ ou un radical -O-COR₉,
- R' et R'' représentent un atome d'hydrogène, un radical alkyle ayant de 1 à 6 atomes de carbone, un radical mono ou polyhydroxyalkyle, un radical aryle éventuellement substitué ou un reste d'amino acide ou de sucre ou encore, pris ensemble, forment un hétérocycle,
- X représente un atome d'oxygène ou de soufre,
- n est un nombre entier compris inclusivement entre 0 et 4,
- m est un nombre entier compris inclusivement entre 0 et 6,
- p est un nombre entier compris inclusivement entre 1 et 3,
- q est un nombre entier compris inclusivement entre 0 et 1,
- t est un nombre entier compris inclusivement entre 0 et 2,
- z est un nombre entier compris inclusivement entre 0 et 2.
ainsi que leurs sels, et leurs analogues chiraux.

2. Composés selon la revendication 1, caractérisés en ce qu'ils se présentent sous forme de sels d'un métal alcalin ou alcalino-terreux, ou encore de zinc ou d'une amine organique.

3. Composés selon l'une des revendications 1 ou 2, caractérisés en ce que les radicaux alkyles ayant de 1 à 6 atomes de carbone sont choisis dans le groupe constitué par les radicaux méthyle, éthyle, isopropyle, butyle, tertiobutyle et hexyle.

4. Composés selon l'une quelconque des revendications précédentes, caractérisés en ce que les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

5. Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

6. Composés selon l'une quelconque des revendications précédentes, caractérisés en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, caractérisés en ce que le radical aryle est un radical phényle éventuellement substitué par au moins un atome d'halogène, un hydroxyle ou une fonction nitro.

8. Composés selon l'une quelconque des revendications précédentes, caractérisés en ce que les radicaux aralkyles sont choisis dans le groupe constitué par les radicaux benzyle ou phénéthyle éventuellement substitués par au moins un atome d'halogène, un hydroxyle ou une fonction nitro.

9. Composés selon l'une quelconque des revendications précédentes, caractérisés en ce que les radicaux alkényles sont choisis dans le groupe constitué par les radicaux contenant de 1 à 5 atomes de carbone et présentant une ou plusieurs insaturations éthyléniques, en particulier le radical allyle.

10. Composés selon l'une quelconque des revendications précédentes, caractérisés en ce que les restes de sucre sont choisis dans le groupe constitué par les restes de glucose, de galactose, de mannose ou d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les atomes d'halogènes sont choisis dans le groupe constitué par le fluor, le chlore et le brome.

15. Composés selon l'une quelconque des revendications précédentes, caractérisés en ce que les radicaux cycloaliphatiques sont parmi les radicaux mono ou polycycliques, en particulier parmi le 1-méthyl cyclohexyle et le 1-adamantyle.

16. Composés selon la revendication 1, caractérisés par le fait qu'ils sont pris dans le groupe constitué par :
- 2-(5,6,7,8-tétrahydro-5,5,8,8-tétraméthyl-2-anthracényl)-4-thiophène carboxylate d'éthyle,
- Acide 2-(5,6,7,8-tétrahydro-5,5,8,8-tétraméthyl-2-anthracényl)-4-thiophène carboxylique,
- 4-(5,6,7,8-tétrahydro-5,5,8,8-tétraméthyl-2-anthracényl)-2-thiophène carboxylate de méthyle,
- Acide 4-(5,6,7,8-tétrahydro-5,5,8,8-tétraméthyl-2-anthracényl)-2-thiophène carboxylique,
- 6-(5,6,7,8-tétrahydro-5,5,8,8-tétraméthyl-2-anthracényl)-nicotinate d'éthyle,
- Acide 6-(5,6,7,8-tétrahydro-5,5,8,8-tétraméthyl-2-anthracényl)-nicotinique.
- Acide N-méthyl-4-(5,6,7,8-tétrahydro-5,5,8,8-tétraméthyl-2-anthracényl)-2-pyrrole carboxylique.
- Acide 4-(5,6,7,8-tétrahydro-5,5,8,8-tétraméthyl-2-anthracényl)-2-pyrrole carboxylique.
- 2-hydroxy-4-(5,6,7,8-tétrahydro-5,5,8,8-tétraméthyl-2-anthracényl)benzoate de méthyle.
- Acide 2-hydroxy-4-(5,6,7,8-tétrahydro-5,5,8,8-tétraméthyl-2-anthracényl) benzoïque.
- Acide 2-méthoxy-4-(5,6,7,8-tétrahydro-5,5,8,8-tétraméthyl-2-anthracényl) benzoïque.
- Acide 2-hydroxy-4-[7-(1-adamantyl)-6-benzyloxy-2-naphtyl]benzoïque.
- Acide 2-hydroxy-4-[7-(1-adamantyl)-6-hydroxy-2-naphtyl]benzoïque.
- Acide 2-hydroxy-4-[7-(1-adamantyl)-6-hexyloxy-2-naphtyl]benzoïque.
- Acide 4-[7-(1-adamantyl)-6-méthoxyéthoxyméthoxy-2-naphtyl]benzoïque.
- Acide 3-méthyl-4-(5,6,7,8-tétrahydro-5,5,8,8-tétraméthyl-2-anthracényl) benzoïque.
- Acide N-propyl-4-(5,6,7,8-tétrahydro-5,5,8,8-tétraméthyl-2-anthracényl)-2-pyrrole carboxylique.
- Acide 2-propyloxy-4-(5,6,7,8-tétrahydro-5,5,8,8-tétraméthyl-2-anthracényl) benzoïque.
- Acide 2-hexyloxy-4-(5,6,7,8-tétrahydro-5,5,8,8-tétraméthyl-2-anthracényl) benzoïque.
- Acide 5-[7-(1-adamantyl)-6-benzyloxy-2-naphtyl]-2-thiophènecarboxylique.
- Acide 4-[7-(1-adamantyl)-6-benzyloxy-2-naphtyl]benzoïque.
- Acide 2-chloro-4-(5,6,7,8-tétrahydro-5,5,8,8-tétraméthyl-2-anthracényl) benzoïque.
- Acide 2-hydroxy-4-[7-(1-adamantyl)-6-(4-fluorobenzyl)oxy-2-naphtyl]benzoïque.
- Acide 2-[7-(1-adamantyl)-6-hydroxy-2-naphtyl]-4-thiophènecarboxylique.
- Acide 2-[7-(1-adamantyl)-6-méthoxy-2-naphtyl]-4-thiophènecarboxylique.
- 4-[7-(1-adamantyl)-6-benzyloxycarbonyl-2-naphtyl]benzoate de méthyle.

17. Composés selon la revendication 1, caractérisés en ce que Ar représente un radical de formule (a) ou (i), R₆ représente un radical -OR₇, R₇ ayant la signification donnée ci-avant, et R₂ et R₃ pris ensemble forment avec le noyau naphtalénique adjacent un cycle à 6 chaînons substitué par des groupes méthyles.

18. Composés selon l'une quelconque des revendications précédentes pour une utilisation comme médicament.

19. Composés selon la revendication 18 pour une utilisation comme médicament destiné au traitement des affections dermatologiques, rhumatismales, respiratoires, cardiovasculaires et ophtalmologiques.

20. Utilisation de l'un au moins des composés définis aux revendications 1 à 17 pour la fabrication d'un médicament destiné au traitement des affections dermatologiques, rhumatismales, respiratoires, cardiovasculires et ophtalmologiques.

21. Composition pharmaceutique, caractérisée par le fait qu'elle comprend, dans un support pharmaceutiquement acceptable, au moins un des composés tels que définis à l'une quelconque des revendications 1 à 17.

22. Composition selon la revendication 21, caractérisée en ce que la concentration en composé(s) selon l'une des revendication 1 à 17 est comprise entre 0,001 % et 5 % en poids par rapport à l'ensemble de la composition.

23. Composition cosmétique, caractérisée par le fait qu'elle comprend, dans un support cosmétiquement acceptable, au moins un des composés tels que définis à l'une quelconque des revendications 1 à 17.

24. Composition selon la revendication 23, caractérisée en ce que la concentration en composé(s) selon l'une des revendications 1 à 17 est comprise entre 0,001 % et 3 % en poids par rapport à l'ensemble de la composition.

25. Utilisation d'une composition cosmétique telle que définie à l'une des revendications 23 ou 24 pour l'hygiène corporelle ou capillaire.

## Claims

1. Polycyclic aromatic compounds, characterized by the fact that they correspond to the following general formula (I): 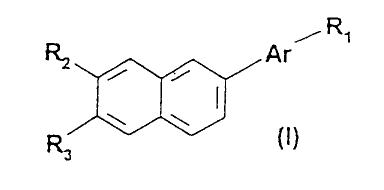 in which:
* R₁ represents:
(i) a hydrogen atom
(ii) a radical -CH₃
(iii) a radical -CH₂OH
(iv) a radical -O-R₄
(v) a radical -S(O)t-R₅
(vi) a radical -CO-R₆
R₄, R₅, R₆ and t having the meaning given below,
* Ar represents a radical chosen from the radicals of the following formulae (a)-(i): 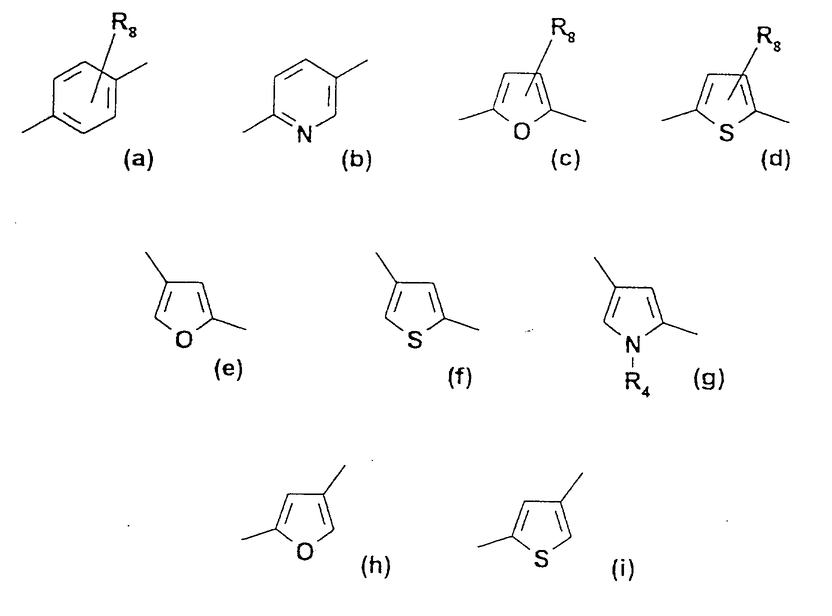 R₄ and R₈ having the meaning given below,
* R₂ represents a linear or branched alkyl radical having 1 to 20 carbon atoms or a cycloaliphatic radical,
* R₃ represents:
(a) a radical -X-(CH₂)ₘ-R₁₀
(b) a radical -(CH₂)ₘ-R₁₁
(c) a radical -CH=CH-(CH₂)ₙ-R₁₁
(d) a radical -O- CH₂-O- CH₂-CH₂ -O- CH₃
X, R₁₀, R₁₁, n and m having the meaning given below, it being understood that in all that precedes:
- R₂ and R₃, taken together, can form with the adjacent naphthalene nucleus a 5- or 6-membered ring optionally substituted by methyl groups and/or optionally interrupted by an oxygen atom or by a radical -S(O)z-, z having the meaning given below:
- R₄ represents a hydrogen atom, an alkyl radical having 1 to 6 carbon atoms or a radical -(CH₂)p-(CO)q-R₅, p, q and R₅ having the meaning given below,
- R₅ represents an alkyl radical having 1 to 6 carbon atoms or a heterocycle,
- R₆ represents:
(a) a hydrogen atom
(b) an alkyl radical having 1 to 6 carbon atoms
(c) a radical of formula: 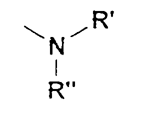 R' and R'' having the meaning given below,
(d) a radical -O-R₇, R₇ having the meaning given below,
- R₇ represents a hydrogen atom, a linear or branched alkyl radical having 1 to 20 carbon atoms, an alkenyl radical, a mono- or polyhydroxyalkyl radical, an aryl or aralkyl radical which is (are) optionally substituted or a sugar residue or an amino acid or peptide residue,
- R₈ represents a halogen atom, an alkyl radical having 1 to 6 carbon atoms, a hydroxyl radical, a radical -OR₉ or -O-COR₉, R₉ having the meaning given below, or alternatively a hydrogen atom when R₂ is the 1-adamantyl radical and R₃ is different from the -OCH₃ radical or from the -OH radical,
- R₉ represents an alkyl radical having 1 to 6 carbon atoms,
- R₁₀ represents a hydrogen atom, an alkyl radical having 1 to 6 carbon atoms, an aryl radical having 1 to 6 carbon atoms, an aralkyl radical, a monohydroxyalkyl radical, a polyhydroxyalkyl radical, a radical -CO-R₆ or alternatively, but only in the case where m is greater than or equal to 2, a radical of formula: 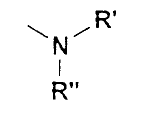 R' and R'' having the meaning given below,
- R₁₁ represents a hydrogen atom, a monohydroxyalkyl radical, a polyhydroxyalkyl radical, a radical -CO-R₆, a radical of formula: 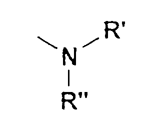 R' and R'' having the meaning given below, or alternatively, but only in the case where m is greater than or equal to 1, a hydroxyl radical, a radical -OR₉ or a radical -O-COR₉,
- R' and R'' represent a hydrogen atom, an alkyl radical having 1 to 6 carbon atoms, a mono- or polyhydroxyalkyl radical, an optionally substituted aryl radical or an amino acid or sugar residue or alternatively, taken together, form a heterocycle,
- X represents an oxygen or sulphur atom,
- n is an integer between 0 and 4 inclusive,
- m is an integer between 0 and 6 inclusive,
- p is an integer between 1 and 3 inclusive,
- q is an integer between 0 and 1 inclusive,
- t is an integer between 0 and 2 inclusive,
- z is an integer between 0 and 2 inclusive,
as well as salts thereof and chiral analogues thereof.

2. Compounds according to Claim 1, characterized in that they are in the form of salts of an alkali or alkaline-earth metal, or alternatively of zinc or of an organic amine.

3. Compounds according to one of Claims 1 or 2, characterized in that the alkyl radicals having 1 to 6 carbon atoms are chosen from the group consisting of methyl, ethyl, isopropyl, butyl, tert-butyl and hexyl radicals.

4. Compounds according to any one of the preceding claims, characterized in that the linear or branched alkyl radicals having 1 to 20 carbon atoms are chosen from the group consisting of methyl, ethyl, propyl, 2-ethylhexyl, octyl, dodecyl, hexadecyl and octadecyl radicals.

5. Compounds according to any one of the preceding claims, characterized in that the monohydroxyalkyl radicals are chosen from the group consisting of 2-hydroxyethyl, 2-hydroxypropyl or 3-hydroxypropyl radicals.

6. Compounds according to any one of the preceding claims, characterized in that the polyhydroxyalkyl radicals are chosen from the group consisting of 2,3-dihydroxypropyl, 2,3,4-trihydroxybutyl or 2,3,4,5-tetrahydroxypentyl radicals or the pentaerythritol residue.

7. Compounds according to any one of the preceding claims, characterized in that the aryl radical is a phenyl radical optionally substituted by at least one halogen atom, a hydroxyl or a nitro functional group.

8. Compounds according to any one of the preceding claims, characterized in that the aralkyl radicals are chosen from the group consisting of benzyl or phenethyl radicals optionally substituted by at least one halogen atom, a hydroxyl or a nitro functional group.

9. Compounds according to any one of the preceding claims, characterized in that the alkenyl radicals are chosen from the group consisting of the radicals containing 1 to 5 carbon atoms and having one or more ethylenic unsaturations, in particular the allyl radical.

10. Compounds according to any one of the preceding claims, characterized in that the sugar residues are chosen from the group consisting of glucose, galactose, mannose or glucuronic acid residues.

11. Compounds according to any one of the preceding claims, characterized in that the amino acid residues are chosen from the group consisting of residues derived from lysine, glycine or aspartic acid.

12. Compounds according to any one of the preceding claims, characterized in that the peptide residues are chosen from the group consisting of dipeptide or tripeptide residues.

13. Compounds according to any one of the preceding claims, characterized in that the heterocyclic radicals are chosen from the group consisting of piperidino, morpholino, pyrrolidino or piperazino radicals which are optionally substituted at position 4 by a C₁-C₆ alkyl radical or a mono- or polyhydroxyalkyl radical.

14. Compounds according to any one of the preceding claims, characterized in that the halogen atoms are chosen from the group consisting of fluorine, chlorine and bromine.

15. Compounds according to any one of the preceding claims, characterized in that the cycloaliphatic radicals are chosen from mono- or polycyclic radicals, in particular from 1-methylcyclohexyl and 1-adamantyl.

16. Compounds according to Claim 1, characterized by the fact that they are chosen from the group consisting of:
- Ethyl 2-(5,6,7,8-tetrahydro-5,5,8,8-tetramethyl-2anthryl)-4-thiophenecarboxylate,
- 2-(5,6,7,8-Tetrahydro-5,5,8,8-tetramethyl-2-anthryl)-4-thiophenecarboxylic acid,
- Methyl 4-(5,6,7,8-tetrahydro-5,5,8,8-tetramethyl-2-anthryl)-2-thiophenecarboxylate,
- 4-(5,6,7,8-tetrahydro-5,5,8,8,-tetramethyl-2-anthryl)-2-thiophenecarboxylic acid
- Ethyl 6-(5,6,7,8-tetrahydro-5,5,8,8-tetramethyl-2-anthryl)nicotinate
- 6-(5,6,7,8-Tetrahydro-5,5,8,8-tetramethyl-2-anthryl)nicotinic acid.
- N-methyl-4-(5,6,7,8-tetrahydro-5,5,8,8-tetramethyl-2-anthryl)-2-pyrrolecarboxylic acid,
- 4-(5,6,7,8-Tetrahydro-5,5,8,8-tetramethyl-2-anthryl)-2-pyrrolecarboxylic acid,
- Methyl 2-hydroxy-4-(5,6,7,8-tetrahydro-5,5,8,8-tetramethyl-2-anthryl)benzoate,
- 2-Hydroxy-4-(5,6,7,8-tetrahydro-5,5,8,8-tetramethyl-2-anthryl)benzoic acid,
- 2-Methoxy-4-(5,6,7,8-tetrahydro-5,5,8,8-tetramethyl-2-anthryl)benzoic acid,
- 2-Hydroxy-4-[7-(1-adamantyl)-6-benzyloxy-2-naphthyl]benzoic acid,
- 2-Hydroxy-4-[7-(1-adamantyl)-6-hydroxy-2-naphthyl]benzoic acid,
- 2-Hydroxy-4-[7-(1-adamantyl)-6-hexyloxy-2-naphthyl]benzoic acid,
- 4-[7-(1-Adamantyl)-6-methoxyethoxymethoxy-2-naphthyl]benzoic acid,
- 3-Methyl-4-(5,6,7,8-tetrahydro-5,5,8,8-tetramethyl-2-anthryl)benzoic acid,
- N-Propyl-4-(5,6,7,8-tetrahydro-5,5,8,8-tetramethyl-2-anthryl)-2-pyrrolecarboxylic acid,
- 2-Propyloxy-4-(5,6,7,8-tetrahydro-5,5,8,8-tetramethyl-2-anthryl)benzoic acid,
- 2-Hexyloxy-4-(5,6,7,8-tetrahydro-5,5,8,8-tetramethyl-2-anthryl)benzoic acid,
- 5-[7-(1-Adamantyl)-6-benzyloxy-2-naphthyl]-2-thiophenecarboxylic acid,
- 4-[7-(1-Adamantyl)-6-benzyloxy-2-naphthyl]benzoic acid,
- 2-Chloro-4-(5,6,7,8-tetrahydro-5,5,8,8-tetramethyl-2-anthryl)benzoic acid,
- 2-Hydroxy-4-[7-(1-adamantyl)-6-(4-fluorobenzyl)oxy-2-naphthyl]benzoic acid,
- 2-[7-(1-Adamantyl)-6-hydroxy-2-naphthyl]-4-thiophenecarboxylic acid,
- Methyl 4-[7-(1-adamantyl)-6-benzyloxycarbonyl-2-naphthyl]benzoate.

17. Compounds according to Claim 1, characterized in that Ar represents a radical of formula (a) or (i), R₆ represents a radical -OR₇, R₇ having the meaning given above, and R₂ and R₃, taken together, form with the adjacent naphthalene nucleus a 6-membered ring substituted by methyl groups.

18. Compounds according to any one of the preceding claims, for use as medicinal products.

19. Compounds according to Claim 18, for use as medicinal products intended for the treatment of dermatological, rheumatic, respiratory, cardiovascular and ophthalmological conditions.

20. Use of at least one of the compounds defined in Claims 1 to 17 for the manufacture of a medicinal product intended for the treatment of dermatological, rheumatic, respiratory, cardiovascular and ophthalmological conditions.

21. Pharmaceutical composition, characterized by the fact that it comprises, in a pharmaceutically acceptable carrier, at least one of the compounds as defined in any one of Claims 1 to 17.

22. Composition according to Claim 21, characterized in that the concentration of compound(s) according to one of Claims 1 to 17 is between 0.001 % and 5 % by weight relative to the whole composition.

23. Cosmetic composition, characterized by the fact that it comprises, in a cosmetically acceptable carrier, at least one of the compounds as defined in any one of Claims 1 to 17.

24. Composition according to Claim 23, characterized in that the concentration of compound(s) according to one of Claims 1 to 17 is between 0.001 % and 3 % by weight relative to the whole composition.

25. Use of a cosmetic composition as defined in one of Claims 23 or 24 for body or hair care.

## Patentansprüche

1. Polycyclische aromatische Verbindungen,
**dadurch gekennzeichnet, daß**
sie der folgenden allgemeinen Formel (I) entsprechen: 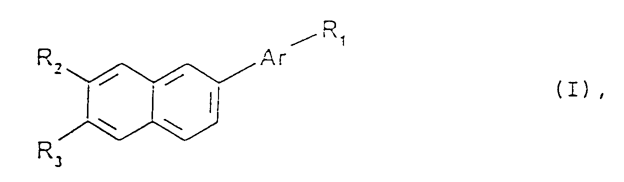 worin bedeuten:
* R₁:
(i) ein Wasserstoffatom,
(ii) die Gruppe -CH₃,
(iii) die Gruppe -CH₂-OH
(iv) eine Gruppe -O-R₄
(v) eine Gruppe -S(O)ₜ-R₅
(vi) eine Gruppe -CO-R₆,
wobei R₄, R₅, R₆ und t die nachstehend angegebenen Bedeutungen aufweisen,
* Ar eine Gruppe, die unter den Gruppen der folgenden Formeln (a) bis (i) ausgewählt ist: 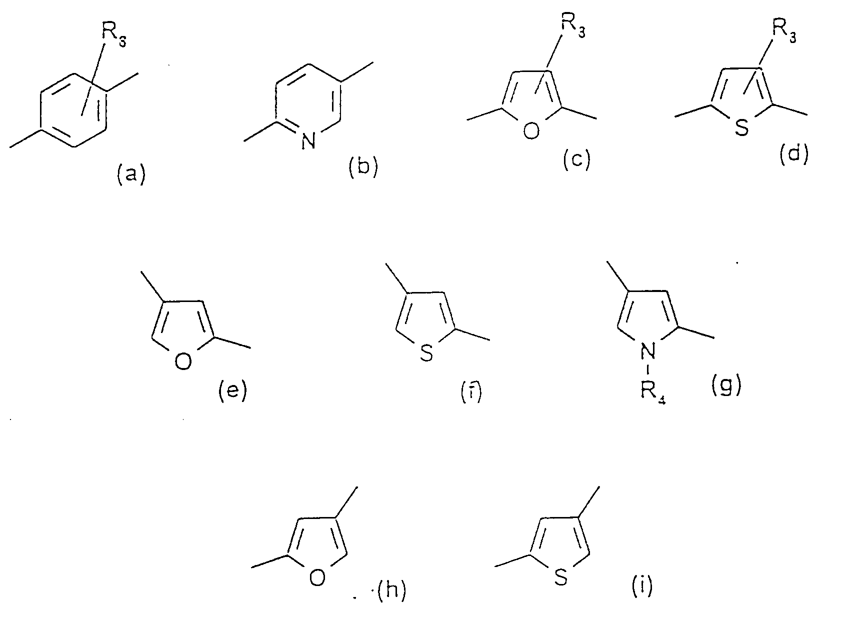 worin R₄ und R₈ die nachstehend angegebenen Bedeutungen aufweisen,
* R₂ eine geradkettige oder verzweigte Alkylgruppe mit 1 bis 20 Kohlenstoffatomen oder eine cycloaliphatische Gruppe,
* R₃
(a) eine Gruppe -X-(CH2)ₘ-R₁₀
(b) eine Gruppe -(CH₂)ₘ-R₁₁
(c) eine Gruppe -CH=CH-(CH₂)ₙ-R₁₁
(d) eine Gruppe -O-CH₂-O-CH₂-CH₂-O-CH₃
wobei X, R₁₀, R₁₁, n und m die nachstehend angebebenen Bedeutungen aufweisen,
mit der Maßgabe, daß im folgenden:
- R₂ und R₃ gemeinsam mit dem angrenzenden Naphthalinring einen 5- oder 6-gliedrigen Ring bilden können, der gegebenenfalls mit Methylgruppen substituiert ist und/oder gegebenenfalls durch ein Sauerstoffatom oder durch eine Gruppe S(O)_{z} unterbrochen ist, wobei z die nachfolgend angegebene Bedeutung aufweist,
- R₄ ein Wasserstoffatom, eine Alkylgruppe mit 1 bis 6 Kohlenstoffatomen oder eine Gruppe -(CH₂)ₚ-(CO)_{q}-R₅ bedeutet, wobei p, q und R₅ die nachfolgend angegebenen Bedeutungen aufweisen,
- R₅ eine Alkylgruppe mit 1 bis 6 Kohlenstoffatomen oder einen Heterocyclus bedeutet,
- R₆ bedeutet
(a) ein Wasserstoffatom
(b) eine Alkylgruppe mit 1 bis 6 Kohlenstoffatomen
(c) eine Gruppe der Formel: 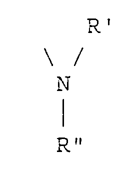 wobei R' und R'' die nachfolgend angegebenen Bedeutungen aufweisen,
(d) eine Gruppe -O-R₇, wobei R₇ die nachfolgend angegebene Bedeutung aufweist,
- R₇ Wasserstoff, eine geradkettige oder verzweigte Alkylgruppe mit 1 bis 20 Kohlenstoffatomen, eine Alkenylgruppe, eine Mono- oder Polyhydroxyalkylgruppe, Aryl- oder Aralkylgruppe, die gegebenenfalls substituiert sind, oder einen Zucker-, Aminosäure- oder Peptidrest bedeutet,
- R₈ ein Halogenatom, eine Alkylgruppe mit 1 bis 6 Kohlenstoffatomen, eine Hydroxygruppe, eine Gruppe -OR₉ oder eine Gruppe -O-COR₉ bedeutet, wobei R₉ die nachfolgend angegebene Bedeutung aufweist, oder auch ein Wasserstoffatom bedeutet, wenn R₂ die gruppe 1-Adamantyl bedeutet und R₃ von -OCH₃ oder -OH verschieden ist.
- R₉ eine Alkylgruppe mit 1 bis 6 Kohlenstoffatomen bedeutet,
- R₁₀ ein Wasserstoffatom, eine Alkylgruppe mit 1 bis 6 Kohlenstoffatomen, eine Arylgruppe, eine Aralkylgruppe, eine Monohydroxyalkylgruppe, eine Polyhydroxyalkylgruppe, eine Gruppe -CO-R₆ oder auch, jedoch nur in dem Fall, daß m größer oder gleich 2 ist, eine Gruppe der Formel 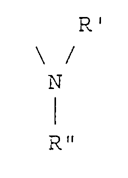 bedeutet, wobei R' und R'' die nachfolgend angegebenen Bedeutungen aufweisen,
- R₁₁ ein Wasserstoffatom, eine Monohydroxyalkylgruppe, eine Polyhydroxyalkylgruppe, eine Gruppe -CO-R₆, eine Gruppe der Formel: 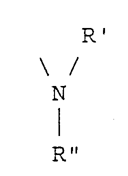 wobei R' und R'' die nachfolgend angegebenen Bedeutungen aufweisen,
oder auch, aber nur in dem Fall, daß m größer oder gleich 1 ist, eine Hydroxygruppe, eine Gruppe -OR₉ oder eine Gruppe -O-COR₉ bedeutet,
- R' und R'' ein Wasserstoffatom, eine Alkylgruppe mit 1 bis 6 Kohlenstoffatomen, eine Mono- oder Polyhydroxyalkylgruppe, eine Arylgruppe, die gegebenenfalls substituiert ist, oder einen Aminosäure- oder Zuckerrest bedeuten oder gemeinsam einen Heterocyclus bilden,
und ferner bedeuten:
- X ein Sauerstoff- oder Schwefelatom,
- n Null oder eine ganze Zahl im Bereich von 1 bis 4,
- m Null oder eine ganze Zahl im Bereich von 1 bis 6,
- p eine ganze Zahl im Bereich von 1 bis 3,
- q Null oder 1,
- t Null, 1 oder 2, z Null, 1 oder 2,
sowie ihre Salze und ihre chiralen Analoga.

2. Verbindungen nach Anspruch 1, dadurch gekennzeichnet, daß sie in Form von Alkali- oder Erdalkalisalzen oder auch Zinksalzen oder Salzen anorganischer Amine vorliegen.

3. Verbindungen nach einem der Ansprüche 1 oder 2, dadurch gekennzeichnet, daß die Alkylgruppen mit 1 bis 6 Kohlenstoffatomen unter den Gruppen Methyl, Ethyl, Isopropyl, Butyl, *tert*.-Butyl und Hexyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter den Gruppen Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter den Gruppen 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter den Gruppen 2,3-Dihydroxypropyl, 2,3,4-Trihydroxybutyl, 2,3,4,5-Tetrahydroxypentyl oder Pentaerythrit ausgewählt sind.

7. Verbindungen nach einem der vorhergehenden Ansprüche, dadurch gekennzeichnet, daß die Arylgruppe eine Phenylgruppe ist, die gegebenenfalls mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter den Gruppen Benzyl oder Phenethyl ausgewählt sind, die gegebenenfalls mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen mit 1 bis 5 Kohlenstoffatomen, die eine oder mehrere ethylenische Doppelbindungen aufweisen, und insbesondere der Allylgruppe ausgewählt sind.

10. Verbindungen nach einem der vorhergehenden Ansprüche, dadurch gekennzeichnet, daß die Zuckerreste unter den Resten von Glucose, Galactose, Mannose und Glucuronsäure ausgewählt sind.

11. Verbindungen nach einem der vorhergehenden Ansprüche, dadurch gekennzeichnet, daß die Aminosäurereste unter den von Lysin, Glycin oder Asparaginsäure abgeleiteten Resten ausgewählt sind.

12. Verbindungen nach einem der vorhergehenden Ansprüche, dadurch gekennzeichnet, daß die Peptidreste unter den Dipeptid- oder Tripeptidresten ausgewählt sind.

13. Verbindungen nach einem der vorhergehenden Ansprüche, dadurch gekennzeichnet, daß die heterocyclischen Gruppen unter den Gruppen Piperidino, Morpholino, Pyrrolidino oder Piperazino ausgewählt sind, die gegebenenfalls in 4-Stellung mit einer C₁₋₆-Alkylgruppe oder Mono- oder Polyhydroxyalkylgruppe substituiert sind.

14. Verbindungen nach einem der vorhergehenden Ansprüche, dadurch gekennzeichnet, daß die Halogenatome unter Fluor, Chlor und Brom ausgewählt sind.

15. Verbindungen nach einem der vorhergehenden Ansprüche, dadurch gekennzeichnet, daß die cycloaliphatischen Gruppen unter den mono- oder polycyclischen Gruppen und insbesondere unter den Gruppen 1-Methylcyclohexyl und 1-Adamantyl ausgewählt sind.

16. Verbindungen nach Anspruch 1, dadurch gekennzeichnet, daß sie aus folgender Gruppe ausgewählt sind:
2-(5,6,7,8-Tetrahydro-5,5,8,8-tetramethyl-2-anthracenyl)-4-thiophencarbonsäureethylester
- 2-(5,6,7,8-Tetrahydro-5,5,8,8-tetramethyl-2-anthracenyl)-4-thiophencarbonsäure
- 4-(5,6,7,8-Tetrahydro-5,5,8,8-tetramethyl-2-anthracenyl)-2-thiophencarbonsäuremethylester
- 4-(5,6,7,8-Tetrahydro-5,5,8,8-tetramethyl-2-anthracenyl)-2-thiophencarbonsäure
- 6-(5,6,7,8-Tetrahydro-5,5,8,8-tetramethyl-2-anthracenyl)-nicotinsäureethylester
- 6-(5,6,7,8-Tetrahydro-5,5,8,8-tetramethyl-2-anthracenyl)-nicotinsäure.
N-Methyl-4-(5,6,7,8-tetrahydro-5,5,8,8-tetramethyl-2-anthracenyl)-2-pyrrolcarbonsäure
4-(5,6,7,8-Tetrahydro-5,5,8,8-tetramethyl-2-anthracenyl)-2-pyrrolcarbonsäure
2-Hydroxy-4-(5,6,7,8-tetrahydro-5,5,8,8-tetramethyl-2-anthracenyl)-benzoesäuremethylester
2-Hydroxy-4-(5,6,7,8-tetrahydro-5,5,8,8-tetramethyl-2-anthracenyl)-benzoesäure
2-Methoxy-4-(5,6,7,8-tetrahydro-5,5,8,8-tetramethyl-2-anthracenyl)-benzoesäure
2-Hydroxy-4-[7-(1-adamantyl)-6-benzyloxy-2-naphthyl]-benzoesäure
2-Hydroxy-4-[7-(1-adamantyl)-6-hydroxy-2-naphthyl]-benzoesäure
2-Hydroxy-4-[7-(1-adamantyl)-6-hexyloxy-2-naphthyl]-benzoesäure
4-[7-(1-Adamantyl)-6-methoxyethoxymethoxy-2-naphthyl]-benzoesäure
3-Methyl-4-(5,6,7,8-tetrahydro-5,5,8,8-tetramethyl-2-anthracenyl)-benzoesäure
N-Propyl-4-(5,6,7,8-tetrahydro-5,5,8,8-tetramethyl-2-anthracenyl)-pyrrolcarbonsäure
2-Propyloxy-4-(5,6,7,8-tetrahydro-5,5,8,8-tetramethyl-2-anthracenyl)-benzoesäure
2-Hexyloxy-4-(5,6,7,8-tetrahydro-5,5,8,8-tetramethyl-2-anthracenyl)-benzoesäure
5-[7-(1-Adamantyl)-6-benzyloxy-2-naphthyl]-2-thiophencarbonsäure
4-[7-(1-Adamantyl)-6-benzyloxy-2-naphthyl]-benzoesäure
2-Chlor-4-(5,6,7,8-tetrahydro-5,5,8,8-tetramethyl-2-anthracenyl)-benzoesäure
2-Hydroxy-4-[7-(1-adamantyl)-6-(4-fluorbenzyl)oxy-2-naphthyl]-benzoesäure
2-[7-(1-Adamantyl)-6-hydroxy-2-naphthyl]-4-thiophencarbonsäure
2-[7-(1-Adamantyl)-6-methoxy-2-naphthyl]-4-thiophencarbonsäure
4-[7-(1-Adamantyl)-6-benzyloxycarbonyl-2-naphthyl]-benzoesäuremethylester

17. Verbindungen nach Anspruch 1, dadurch gekennzeichnet, daß Ar eine Gruppe der Formel (a) oder (i) und R₆ eine Gruppe -OR₇ bedeutet, wobei R₇ die oben angegebene Bedeutung aufweist, und R₂ und R₃ gemeinsam mit dem angrenzenden Naphthalinring einen 6-gliedrigen Ring bilden, der gegebenenfalls mit Methylgruppen substituiert ist.

18. Verbindungen nach einem der vorhergehenden Ansprüche für eine Verwendung als Arzneimittel.

19. Verbindungen nach Anspruch 18 für eine Verwendung als Arzneimittel, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

20. Verwendung mindestens einer der Verbindungen nach den Ansprüchen 1 bis 17 zur Herstellung eines Arzneimittels, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist.

21. Pharmazeutische Zusammensetzung, dadurch gekennzeichnet, daß sie in einem pharmazeutisch akzeptablen Träger mindestens eine Verbindung nach einem der Ansprüche 1 bis 17 enthält.

22. Zusammensetzung nach Anspruch 21, dadurch gekennzeichnet, daß die Konzentration der Verbindung(en) nach einem der Ansprüche 1 bis 17 im Bereich von 0,001 bis 5 Gew.-%, bezogen auf die gesamte Zusammensetzung, liegt.

23. Kosmetische Zusammensetzung, dadurch gekennzeichnet, daß sie in einem kosmetisch akzeptablen Träger mindestens eine Verbindung nach einem der Ansprüche 1 bis 17 enthält.

24. Zusammensetzung nach Anspruch 23, dadurch gekennzeichnet, daß die Konzentration der Verbindung(en) nach einem der Ansprüche 1 bis 17 im Bereich von 0,001 bis 3 Gew.-%, bezogen auf die gesamte Zusammensetzung, liegt.

25. Verwendung einer kosmetischen Zusammensetzung nach einem der Ansprüche 23 oder 24 zur Körper- oder Haarhygiene.
